# EUROPEAN PATENT APPLICATION

(11) **EP 4 588 520 A2**
(43) Date of publication of application: **23.07.2025**
(21) Application number: 24206840.1
(22) Date of filing: 22.06.2020
(51) Int. Cl.: A61P 29/00

(54) **COMPOSITION MODULATING BOTULINUM NEUROTOXIN EFFECT**

(30) Priority: 21.06.2019 EP 19181635
(62) Divisional of application: 20734883.0
(71) Applicant: Fastox Pharma SA, 1003 Lausanne (CH)
(72) Inventor: LE DOUSSAL, Jean-Marc, 1007 Lausanne (CH); CROS, Cécile, 74580 Viry (FR); HULO, Nicolas, 1252 Meinier (CH); MACHICOANE, Mickaël, 1290 Versoix (CH)
(74) Representative: KATZAROV S.A.

(57) **Abstract**

The present invention relates to a method for modulating the effect of a botulinum neurotoxin composition, that is accelerating the onset of action and /or extending the duration of action and/or enhancing the intensity of action of a botulinum neurotoxin composition, comprising adding at least one postsynaptic inhibitor of cholinergic neuronal transmission to the botulinum neurotoxin composition. The invention also relates to compositions comprising at least one postsynaptic inhibitor of cholinergic neuronal transmission and a botulinum neurotoxin, and their uses for treating aesthetic or therapeutic conditions.

## Description

The present patent application claims the priority of the European patent application EP EP19181635.4 filed on June 21, 2019, which is herein incorporated by reference.

### FIELD OF THE INVENTION

The present invention relates to a composition comprising at least one postsynaptic inhibitor of cholinergic neuronal transmission and a botulinum neurotoxin, and its use for various therapeutic and/or aesthetic purposes. Further, the compositions and methods in which these compositions are used provide advantageous treatments which result in enhancing the effect of botulinum neurotoxin, e.g. accelerating its onset of action and/or extending its duration of action and/or enhancing its intensity of action.

### BACKGROUND OF THE INVENTION

### BoNT structure

Botulinum neurotoxins are toxins impairing neuronal transmission that are preferentially active on cholinergic neurons; one of the main consequences of botulinum neurotoxin action is thus muscle relaxation due to decreased neuronal stimulation. Botulinum neurotoxins are produced as complexes by an anaerobic and spore-forming bacterium *Clostridium botulinum* and to a lesser extent by other Clostridium species, such as *C*. *butyricum, C. barati, C. sporogenes* and *C*. *argentinense.* Eight different serotypes of botulinum neurotoxins known as type A (BoNT/A), B (BoNT/B), C (BoNT/C), D (BoNT/D), E (BoNT/E), F (BoNT/F), G (BoNT/G), and X (BoNT/X) were identified. There are currently 8, 8, 12 and 9 known BoNT/A, BoNT/B, BoNT/E and BoNT/F subtypes respectively. Types A, B, E and F are toxic for human beings while types C, D and G more often cause toxicity for example in birds, horses, cattle and primates. Type X has only been described recently (Zhang et al., Nature Communications, volume 8, 14130 (2017)), after a case report of infant botulism in Japan in 1995.

Botulinum neurotoxin complexes are present in form of high-molecular weight protein complexes comprising two components; namely the enzymatically active neurotoxin component and an associated non-toxic bacterial protein component which can be regarded as a coat protein including hemagglutinin and non-hemagglutinin proteins. The molecular weight of the botulin toxin complexes varies among the distinct botulinum toxin serotypes from about 300 kDa to about 900 kDa; botulinum neurotoxins devoid of the non-toxic bacterial protein component has a molecular weight of about 150 kDa. Regarding the aesthetic or therapeutic application, the coat protein is reported to have no significant function and does not contribute to the neurotoxic properties. The neurotoxin component is expressed as an inactive single-chain precursor having a molecular weight for all of the known botulinum neurotoxin serotypes of about 150 kDa. This single chain precursor is activated by proteolytic cleavage to generate a disulfide-linked two-chain protein. The about 50 kDa light chain protein contains the catalytic domain and is a zinc-containing metalloprotease and acts as a zinc-endopeptidase. The about 100 kDa heavy chain protein comprises a translocation domain and a receptor-binding domain. The heavy chain mediates binding to the presynaptic cholinergic nerve terminals, in particular to the presynaptic part of the motor end plate or neuromuscular junction, and internalization of the neurotoxin into the cell.

### BoNT mechanism of action

When reaching the effector tissue, typically the neuromuscular junction, the botulinum neurotoxin heavy chain mediates the preferential uptake by cholinergic neurons, thanks to its double binding to the presynaptic receptors (Synaptic Vesicle 2 proteins) and to specific gangliosides. Subsequently, the botulinum neurotoxin can enter the neuronal cell via receptor-mediated endocytosis and stays inside the endocytic vesicles of the neuronal cells. Upon acidification of the vesicle, the light chain is translocated into the cytoplasm and split off. The light chain toxic moiety is able to cleave one or more of the proteins that form the SNARE protein complex (i.e. SNAP-25, syntaxin and VAMP), depending on the BoNT serotype. The SNARE complex normally enables membrane fusion between vesicles and the plasma membrane, thereby allowing the neurotransmitter acetylcholine to leave the cell. For instance, by discharging the neurotransmitter acetylcholine in the synaptic cleft, a nerve impulse is transmitted to the muscle, which signals the muscle to contract. As the formation of the SNARE complex is prevented through the cleavage of a protein essentially to form said complex by for example BoNT/A, neurotransmitter exocytosis is blocked, and notably, the acetylcholine release is stopped. Consequently, the transmission between the nerve and the muscle (or other effector tissues) is blocked. Notably, the catalytically active botulinum neurotoxin light chain is able to persist in neuronal cells for several weeks, allowing a long-term yet reversible inhibition of the nerve transmission.

### BoNT medical use

The natural property of botulinum neurotoxins to reversibly block the release of acetylcholine from the presynaptic nerve terminals in muscles and other effector tissues such as the secretory glands in the orthosympathetic and parasympathetic systems makes them an important therapeutic option in numerous fields to control muscle (and other effector tissues) overstimulation, and associated pain. For example, BoNT/A is currently used therapeutically in the fields of: movement disorders, especially for managing spasticity and dystonia, where BoNT/A is well-described to efficiently relief dystonia-related pain; urinary disorders, notably overreactive bladder; secretory disorders, namely hyperhidrosis and sialorrhea. Moreover, botulinum neurotoxins are now currently used in a variety of aesthetic indications, such as smoothing facial lines or reducing glabellar, frown and periorbital lines.

### BoNT drugs

Despite sharing a similar metalloprotease activity involved in the cleavage of proteins of the SNARE complex, botulinum neurotoxins may have different onset and/or duration of action depending on their serotypes. For example, whereas BoNT/A and BoNT/F induces a complete localized paralysis in the hind leg of mice within 2 days, BoNT/E produces the same effect within 24 hours. Nevertheless, the duration of BoNT/A-induced neuromuscular paralysis is 28 days in mice, compared to only 5 and 8 days for toxins E and F respectively. A similar pattern has been observed in human, although at a different timescale: BoNT-A paralysis is generally observed within a week and can last for 3 to 4 months (Davletov et al., TRENDS in Neurosciences 28 (2005); pp. 446-452). On the contrary, BoNT-E shows faster onset (24 hours) but much shorter duration (less than 1 month) in human (Yoelin et al., Plast Recontr Surg 142 (2018); pp. 847e-855e).

Because of its unique profile, and activity in human, most attention has been focused on BoNT/A. Since 1991, several commercial botulinum neurotoxins of type A were approved by the U.S. Food and Drug Administration (FDA). The available forms of BoNT/A are Botox^{®}/Vistabel^{®} (Allergan), Dysport^{®}/Azzalure^{®} (Ipsen Biopharm) and Xeomin^{®}/Bocouture^{®} (Merz Pharmaceuticals) among others. The only other BoNT serotype that is currently commercially available is BoNT/B Myobloc^{®}/Neurobloc^{®} (Solstice Neurosciences); its medical/aesthetic use is anecdotal, though. Today, improving BoNT characteristics is still an important goal to reach, in order to better answer medical needs.

### BoNT limiting characteristics

Onset of action, duration of action and intensity of action of BoNTs are key characteristics affecting their utility as pharmaceuticals.

### Onset of action

In aesthetic indications, reduction of wrinkles is generally observable 2 to 3 days after BoNT/A injection while complete blockade (e.g. preventing frowning) needs about 2-3 weeks). Faster onset is often required by the treated subjects. Similarly, in therapeutic uses of BoNT/A such as the treatment of muscle disorders, a faster onset of BoNT action would be clearly beneficial to the patient. On the practitioner standpoint, having a faster onset of muscle paralysis would facilitate the monitoring of the patient: ideally, injection and monitoring should be done the same day.

### Duration of action

Extending the duration of action of BoNTs is desirable to space out injections while keeping the effect. First to limit the number of injections that are a physical and economic burden for patients. Also to limit the risk of developing neutralizing antibodies to the BoNT that may lower the efficacy of further BoNT-based treatment, thus limiting aesthetic or therapeutic options for the subject. Closely repeated injections of BoNT are thus avoided, but this can lead to periods when BoNT is not active in the subject, resulting in patient compliance issues.

### Intensity of action

Enhancing the intensity of action of BoNT can improve the aesthetic result and the relief of disabling and painful conditions. Nevertheless, increasing the injected dose of BoNT may lead to systemic toxicity by BoNT diffusion distant from the injection site. A method to increase the local efficacy of BoNT without increasing its systemic toxicity in proportion would be beneficial.

### Need for improvement

There is therefore a need for a composition allowing to overcome these shortcomings, and in particular for a composition comprising a botulinum neurotoxin that exhibits a faster onset and/or an extended duration of action and/or an enhanced intensity of action of the botulinum neurotoxin.

### Enhancing BoNT effect

### BoNT molecule

As naturally occurring BoNTs show limitations, a first strategy to enhance the effect of these neurotoxins is to modify the protein itself; current recombinant technology offers the possibility of optimizing the characteristics of BoNT through the introduction of modifications to its sequence. The main approach consists in combining heavy and light chains from different BoNTs, resulting in the production of chimeric neurotoxins. The numerous attempts made during the last decades have demonstrated that BoNT effect modulation was indeed feasible; however, the optimized characteristics of chimera are often close to the characteristics of one of the parent BoNT. For instance, Wang et al. (J Biol Chem. 2008 Jun 20;283(25):16993-7002) described chimeric neurotoxins made of BoNT/A and BoNT/E. Noteworthy, AE chimera (comprising light chain from BoNT/A and heavy chain from BoNT/E) behaves similarly to BoNT/A, although exhibiting a slightly enhanced intensity of action and a slightly extended duration of action. Reciprocally, EA chimera (comprising light chain from BoNT/E and heavy chain from BoNT/A) behaves similarly to BoNT/E.

Besides, alternative approaches aim at combining BoNT/A with BoNT/B in order to widen the range of tissues that could be targeted by the neurotoxin. Recent articles (Wang et al., Biochem J. 2012 May 15;444(1):59-67) and international applications (WO 2017/191315 A1) describe such chimeras.

In conclusion, previous attempts of molecular engineering of BoNT proteins turned out to modulate only slightly the characteristics of BoNTs. Alternative approaches have thus been sought after.

### BoNT Delivery

To exert their effect, BoNTs first have to reach the target cell, namely the cholinergic neuron. Shortening the time required for BoNT to spread to the target tissue, or increasing the duration and amount of BoNT present at the synaptic level after administration thus appear as interesting strategies to modulate BoNT effect. Such approaches have been explored by Revance Therapeutics, that developed formulation of BoNT/A including peptides related to the cell penetrating-peptide family. Several patents and patent applications have described this formulation (i.e. WO 2002/07773 or WO 2005/120546). Strikingly, according to experiments on animal model, the formulation seems to limit diffusion and enhance stability of BoNT/A (Stone, et al., Toxicon. 2011 Aug;58(2):159-67).

Clinical trials aimed to demonstrate the enhanced efficacy of this formulation, by comparing it to BoNT/A not associated with the peptide (unformulated). However, investigators have used higher doses of the formulation than unformulated BoNT/A (Carruthers et al., Dermatol Surg. 2017 Nov;43(11):1321-1331); the extended duration of action observed with the formulation might thus be attributed to the increase in dosage only.

Finally, a recent review (Hallett, M., Toxicon. 2015 December 1; 107(0 0): 64-67) highlighted the low influence of diffusion on BoNT uptake. According to latest observations, BoNT delivery to target tissue is mostly driven by convection (subsequent to injection).

In conclusion, strategies aiming at modulating BoNT delivery may have limited potential to enhance BoNT effects. Alternative approaches are thus still needed to achieve this goal.

### BoNT biology

Another strategy to enhance BoNT effect might be to use other molecules that interact with BoNT mechanism of action, such as BoNT entry in neurons, light chain translocation into neuron cytosol, etc.

Because the different BoNTs share some common mechanism of action, it was tempting to try combine BoNT subtypes with different characteristics, for instance to combine the faster onset of action of the BoNT/E with the longer duration of action of the BoNT/A in order to obtain at least an additive effect. Notably, preclinical experiments (Meunier et al., Molecular and Cellular Neuroscience 22 (2003); pp. 454-466) showed that co-injection of BoNT/A and BoNT/E induced: i) a short onset (similar to BoNT/E alone), and ii) a low duration of action (similar to BoNT/E alone). Similar results were obtained in humans wherein BoNT injections were carried out in the *extensor digitorum brevis* (EDB) muscle of the feet in human. Strikingly, double injected BoNT/A and BoNT/E EDB muscles recover their functionality with a time course similar to that of BoNT/E injected muscles (Eleopra et al., Neuroscience Letters 256 (1998); pp. 135-138). All these results demonstrate that combinations of two BoNTs do not provide the effect that would be expected from the mere addition of the effects of each BoNTs administered independently. It is now understood that some BoNTs have dominant effect compared to others, thereby preventing the use of BoNT combinations with additive properties. Therefore, mixtures of BoNTs are unlikely therapeutic candidate to improve the pharmacologic profile of the currently widely used BoNT/A.

Other candidates to interact with BoNT mechanism of actions are neurotoxins targeting the same presynaptic regions as BoNTs. Such toxins exist in the animal kingdom, such as snake and spider neurotoxins, which can reversibly paralyze motor axon terminals with outcomes similar to these observed for BoNT. However, their size and mechanisms of action are quite different, leading to a quite different onset and duration of neuromuscular junction blockade. Generally, unlike BoNTs, these presynaptic neurotoxins cause an acute and quick degeneration of motor axon terminals, followed by a rapid recovery. Again, it is tempting to imagine the therapeutic potential of combinations between such presynaptic neurotoxins and BoNTs. Surprisingly, when injected in muscles previously paralyzed with BoNT/A or BoNT/B, a presynaptic neurotoxin accelerates the recovery of neurotransmission (Duregotti et al., Toxins 7 (2015), pp. 5322-5336).

Other candidates to interact with BoNT mechanism of action would be molecules modulating the activity of the presynaptic neurons. Noteworthy, Thyagarajan et al. (J Pharmacol Exp Ther. 2009 Nov;331(2):361-71) reported that the activation of TRPV1 channel appeared to inhibit BoNT entry into presynaptic neurons, thereby preventing BoNT myorelaxation; TRPV1, a calcium channel expressed by presynaptic neurons, was activated in this study by the use of capsaicin. Although the principle that was demonstrated is interesting, the only observed effect was an inhibition of BoNT effect: no molecule enhancing BoNT actions was described.

Altogether, these results demonstrate that the therapeutic potential of combinations between BoNT and presynaptically active molecules is very limited.

Interestingly, there is no report to date of the use of BoNT combined with molecules acting at the postsynaptic level for enhancing its effect.

### SUMMARY OF THE INVENTION

The inventors have now identified a method for enhancing BoNT effect by combining BoNT with Postsynaptic inhibitors of Neuronal Transmission (PoNT) targeting i) the skeletal muscles, ii) the smooth muscles, iii) the cardiac muscles, iv) the secretory glands.

Consequently, the present invention relates to a method for enhancing the effect of a botulinum neurotoxin composition, comprising the addition of at least one postsynaptic inhibitor of cholinergic neuronal transmission to the botulinum neurotoxin composition.

The effect of a botulinum neurotoxin composition can be enhanced by accelerating the onset of action and/or extending the duration of action and/or enhancing the intensity of action of a botulinum neurotoxin.

In another aspect, the present invention provides a composition comprising at least one postsynaptic inhibitor of cholinergic neuronal transmission (PoNT) and a botulinum neurotoxin (BoNT).

PoNTs used in the composition of the invention are postsynaptic small molecules and/or postsynaptic peptides that specifically bind to postsynaptic receptors expressed by effectors tissues such as muscles or secretory glands.

Botulinum neurotoxin used in the composition of the invention is type A, B, E, or a combination of heavy and light chains of type A, B, E botulinum neurotoxin.

Postsynaptic receptors expressed by a skeletal muscle cell are selected from the group comprising or consisting of (α1)₂β1δε nAChr, (α1)₂β1δy nAChr, RyR1, CaV1.1 and Nav1.4.

Postsynaptic receptors expressed by a smooth muscle cell are selected from the group comprising or consisting of M3 mAChrs, RyR2, CaV1.2 and Nav1.5.

Postsynaptic receptors expressed by a cardiac muscle cell are selected from the group comprising or consisting of M2 mAChr, RyR2, CaV1.1, CaV1.2 and Nav1.5.

Postsynaptic receptors expressed by a secretory gland cell are selected from the group comprising or consisting of M1 mAChR, M3 mAChR, α₁ adrenergic receptor and β1 adrenergic receptor.

The composition of the invention not only combines a rapid onset of action of PoNTs with a prolonged duration of action of BoNT, but also PoNTs synergistically potentiates the onset of action, the duration of action, and the intensity of action of BoNT.

In all embodiments, the at least one postsynaptic inhibitor of cholinergic neuronal transmission is compatible with the late onset of botulinum neurotoxin.

In a particular embodiment, the at least one postsynaptic inhibitor of cholinergic neuronal transmission is a fast onset postsynaptic peptide and/or a fast onset postsynaptic small molecule.

The composition of the invention can furthermore be used in aesthetic treatment, such as for example reducing wrinkles, lines, such as glabellar lines, or furrows, muscle volume for aesthetic purposes (such as masseter or calf), hypertrophic scars and other dermatological conditions.

The composition of the invention can furthermore be used in therapeutic treatment, such as for example the treatment of (i) skeletal muscle disorders comprising movement disorder, dystonia, cervical dystonia, spasmodic torticollis, focal dystonia, focal hand dystonia, blepharospasm, eyelid disorder, strabismus, spasticity, cerebral palsy, focal spasticity, limb spasticity, spasms, hemifacial spasm, tremors, tics, bruxism, apraxia and freezing of gait and (ii) of the pain associated to these disorders.

The composition of the invention can furthermore be used in the treatment of (i) smooth muscle disorders comprising spasmodic dysphonia, laryngeal dystonia, oromandibular dysphonia, lingual dystonia and other voice disorders, achalasia, dysphagia, esophagia, gastroparesis, spasmodic colitis, neurogenic bladder, overreactive bladder, interstitial cystitis, benign prostatic hyperplasia, urinary dysfunction, fecal incontinence, constipation, anismus, anal fissure, uterine pain (dysmenorrhea, dyspareunia), vaginal pain (vaginismus, vulvodynia), pelvic pain, ischiocavernous muscle (priapism), other muscle tone disorders and other disorders characterized by involuntary movements of muscle groups and (ii) of the pain associated to these disorders.

The composition of the invention can furthermore be used in the treatment of cardiac disorder comprising atrial fibrillation.

The composition of the invention can furthermore be used in the treatment of secretory gland disorder comprising lacrimation, hyperhidrosis (hand, foot and armpit), sialorrhea, excessive salivation, excessive gastrointestinal secretions, excessive production of sebaceous glands (and related conditions such as acne) and secretory disorders.

In all embodiments, the PoNT and the BoNT must be administered in combination, i.e. simultaneously or within a time schedule allowing their simultaneously effective presence at the synapse level. In other words, the at least one postsynaptic inhibitor of cholinergic neuronal transmission (PoNT) and a botulinum neurotoxin may be administered concomitantly, separately or staggered in time, with the proviso that the botulinum neurotoxin is administered before the decay of PoNT activity occurs, and optionally the botulinum neurotoxin is administered after the onset of PoNT activity.

Finally, the present invention relates to a kit implementing the methods of the invention, said kit comprising a needle and a corresponding syringe, at least one postsynaptic inhibitor of cholinergic neuronal transmission and a botulinum neurotoxin.

The present invention and its preferred embodiments are described in further details below.

### BRIEF DESCRIPTION OF THE FIGURES

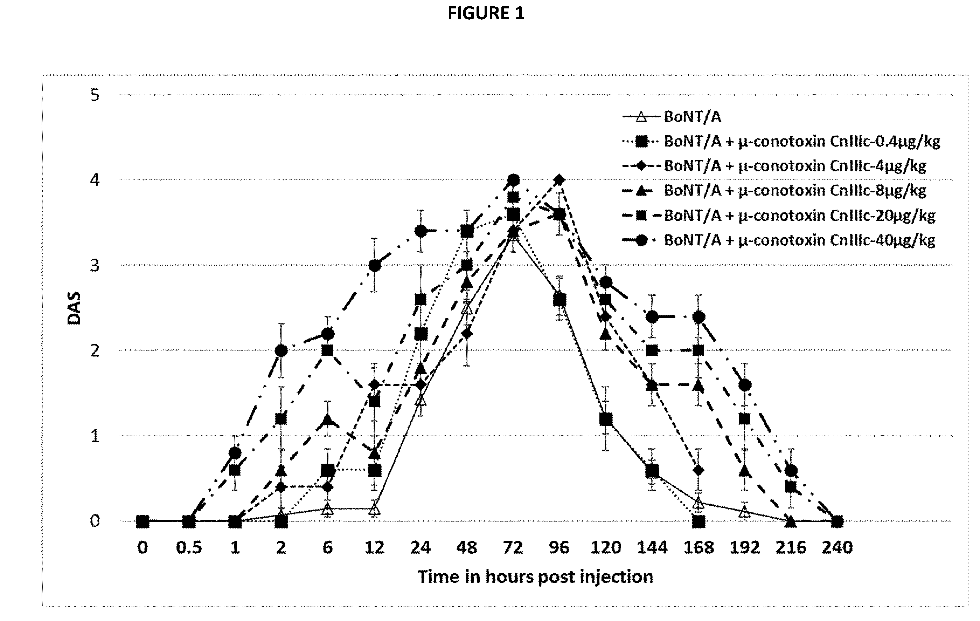

**Figure 1****:** DAS assay - Groups of 5 rats were injected into the right tibialis anterior muscle with 5 U/kg of BoNT/A, or 5 U/kg of BoNT/A combined with increasing amounts of µ-conotoxin Cnlllc peptide ranging from 0.4 to 40 µg/kg; and Digit Abduction Scores (DAS) were assessed for up to 10 days. The mean DAS and standard error of the mean for the groups at each time point are shown. Rats injected into the left tibialis anterior muscle with saline as a control exhibited a DAS of 0 throughout the study period and data is thus not shown on the graph.

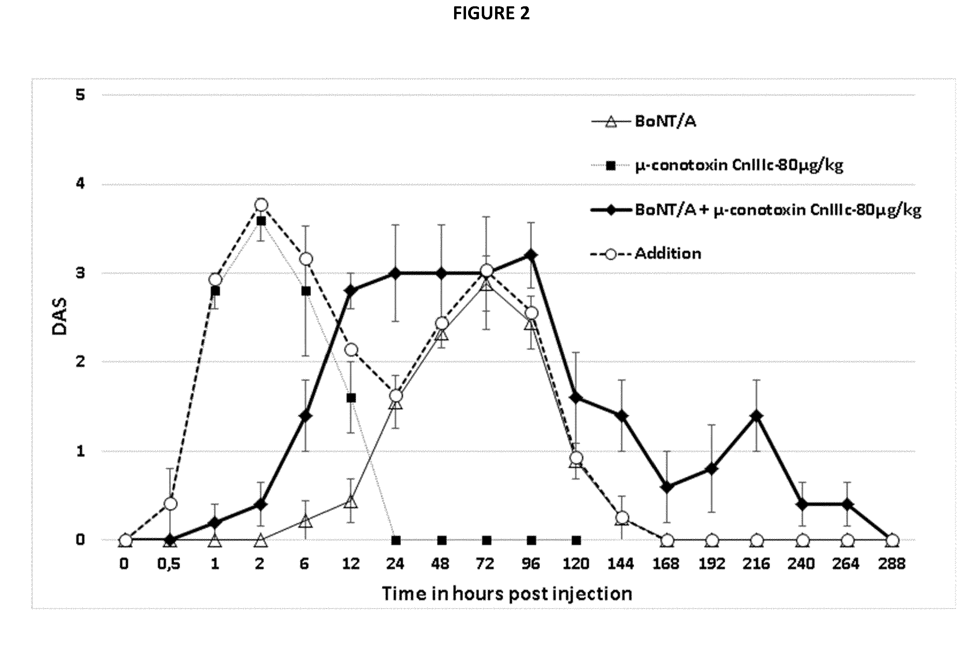

**Figure 2****:** DAS assay - Groups of 5 rats were injected into the right tibialis anterior muscle with 5 U/kg of BoNT/A, 80 µg/kg of µ-conotoxin Cnlllc, 5 U/kg of BoNT/A combined with 80 µg/kg of µ-conotoxin Cnlllc; and DAS were assessed for up to 12 days. Then, data of the first two conditions (5 U/kg of BoNT/A, 80 µg/kg of µ-conotoxin Cnlllc) were added and plotted ("Addition"). Rats injected into the left tibialis anterior muscle with saline as a control exhibited a DAS of 0 throughout the study period and data is thus not shown on the graph.

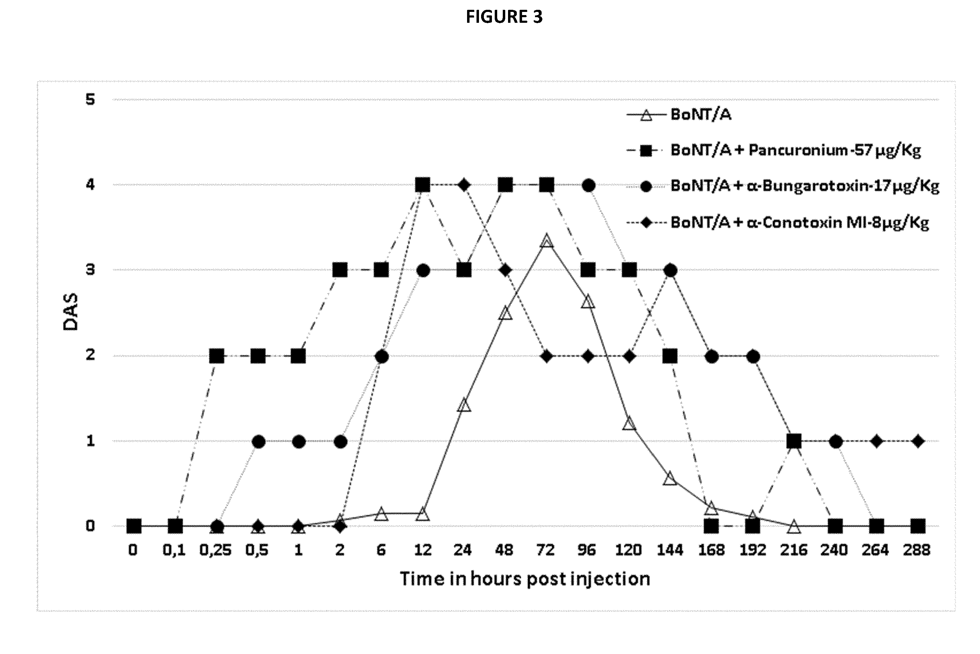

**Figure 3****:** DAS assay - Groups of 1 rat were injected into the right tibialis anterior muscle with 5 U/kg of BoNT/A, 5 U/kg of BoNT/A combined with 57 µg/kg of pancuronium, 5 U/kg of BoNT/A combined with 8 µg/kg of α-conotoxin MI peptide, 5 U/kg of BoNT/A combined with 17 µg/kg of α-bungarotoxin peptide; and DAS were assessed for up to 12 days. Rats injected into the left tibialis anterior muscle with saline as a control exhibited a DAS of 0 throughout the study period and data is thus not shown on the graph.

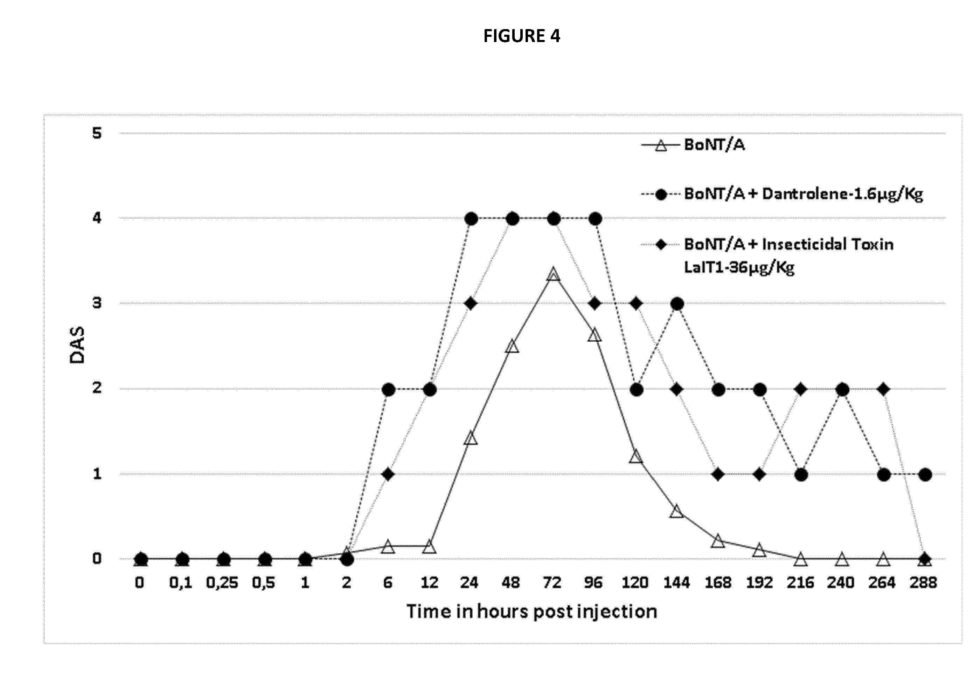

**Figure 4****:** DAS assay - Groups of 1 rat were injected into the right tibialis anterior muscle with 5 U/kg of BoNT/A, 5 U/kg of BoNT/A combined with 1.6 µg/kg of dantrolene, 5 U/kg of BoNT/A combined with 36 µg/kg of Insecticidal Toxin LalT1; and DAS were assessed for up to 12 days. Rats injected into the left tibialis anterior muscle with saline as a control exhibited a DAS of 0 throughout the study period and data is thus not shown on the graph.

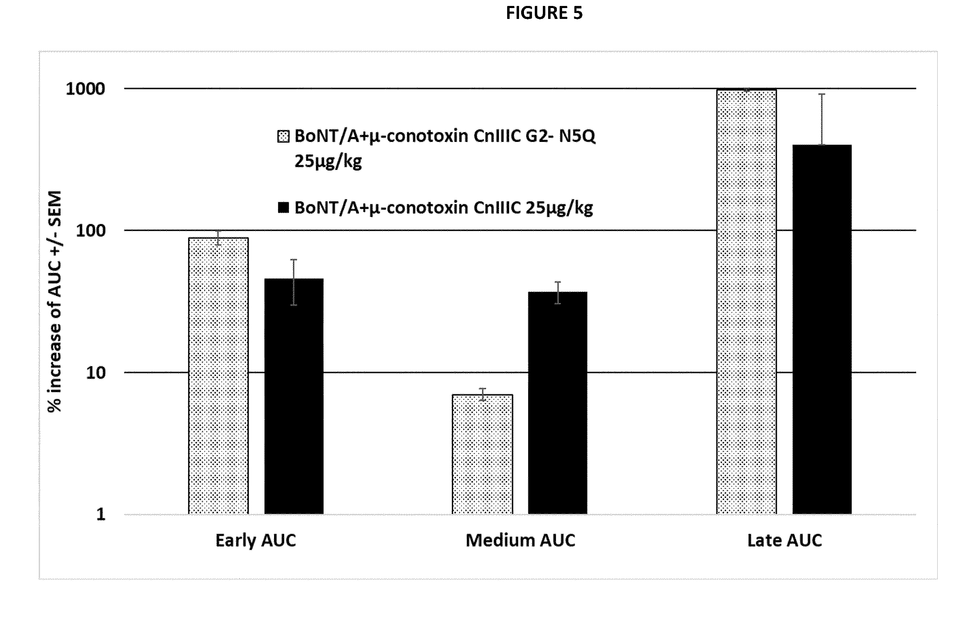

**Figure 5****:** Synergistic effect of the combinations of 5U/kg of BoNT/A with 25 µg/kg of either wild type type µ-conotoxin Cnlllc peptide (SEQ ID NO:54) or 25 µg/kg of a variant of type µ-conotoxin Cnlllc peptide (SEQ ID NO:95) were analyzed using calculation of Areas Under the Curve (AUC) during the early, medium and late phase of the effect.

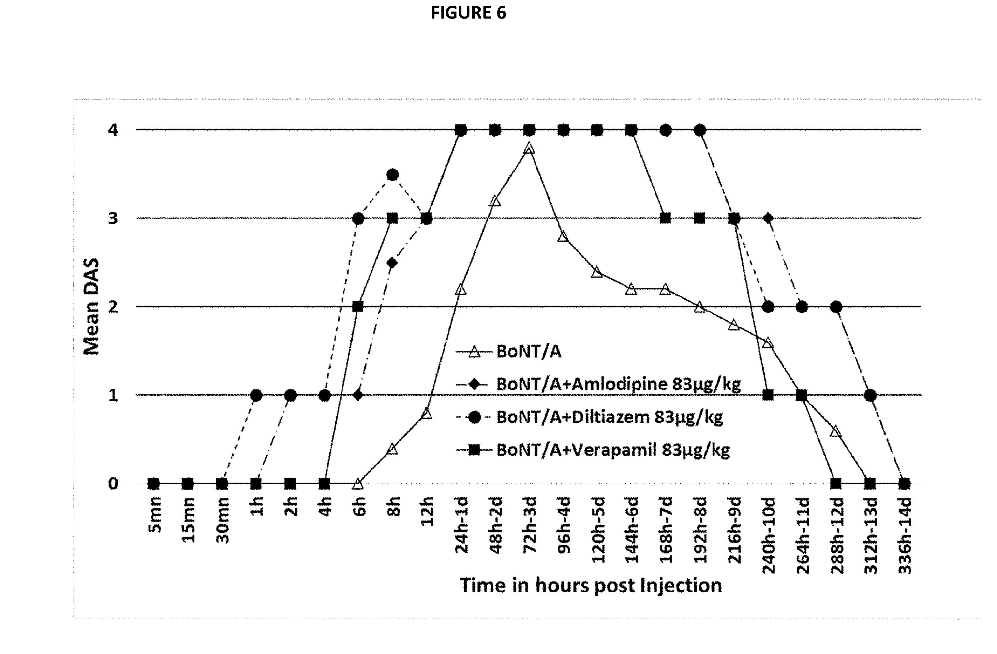

**Figure 6****:** DAS assay - Groups of 5 rats were injected into the right tibialis anterior muscle with 5 U/kg of BoNT/A, 5 U/kg of BoNT/A combined with 83 µg/kg of amlodipine, 5 U/kg of BoNT/A combined with 83 µg/kg of diltiazem and 83 µg/kg of verapamil; and DAS were assessed for up to 14 days. Rats injected into the left tibialis anterior muscle with saline as a control exhibited a DAS of 0 throughout the study period and data is thus not shown on the graph.

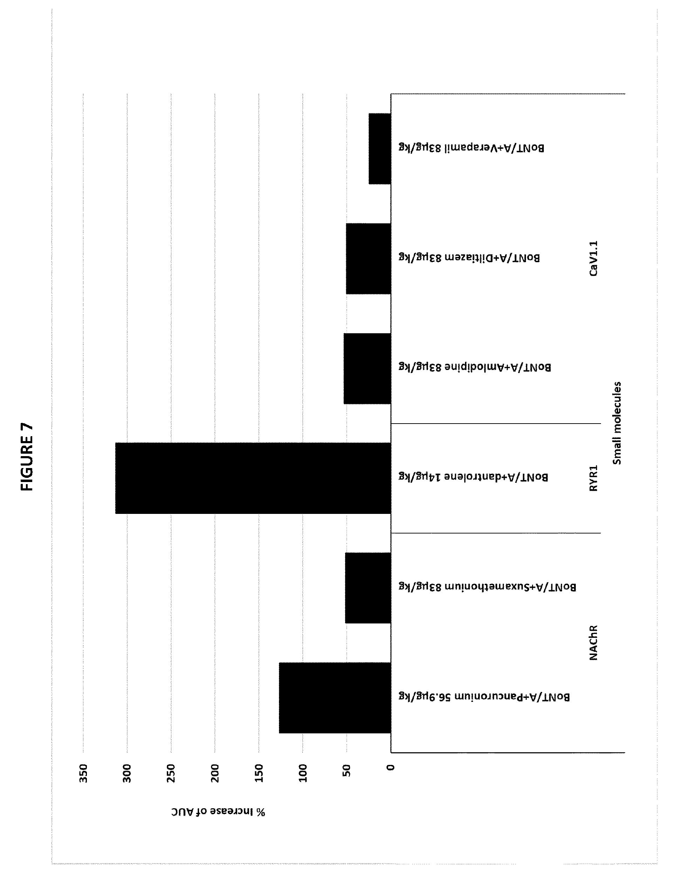

**Figure 7****:** Synergistic effect of the combinations of 5U/kg of BoNT/A with small molecules that specifically inhibits NAChR receptors (56.9 µg/kg pancuronium and 83 µg/kg suxamethonium), RYR1 receptors (14 µg/kg dantrolene) or CaV1.1 receptors (83 µg/kg amlodipine, 83 µg/kg diltiazem and 83 µg/kg verapamil) were analyzed using calculation of total Areas Under the Curve (AUC).

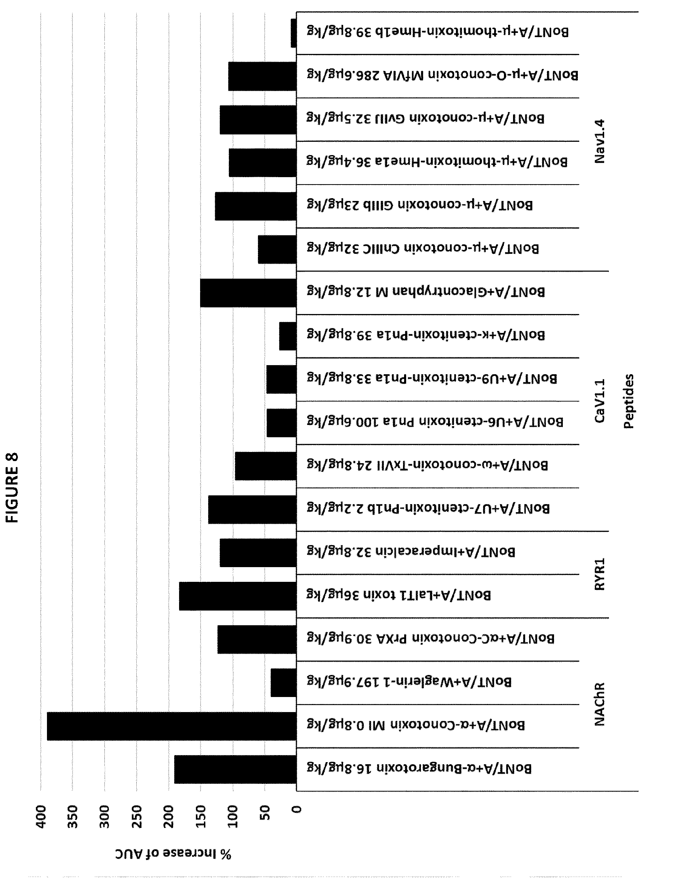

**Figure 8****:** Synergistic effect of the combinations of 5U/kg of BoNT/A with peptides that specifically inhibit NAChR receptors (16.8 µg/kg α-Bungarotoxin, 0.8 µg/kg α-conotoxin MI, 197.9 µg/kg Waglerin-1, 30.9 µg/kg αC-Conotoxin PrXA), RYR1 receptors (36 µg/kg LalT1 toxin, 32.8 µg/kg Imperacalcin), CaV1.1 receptors (2.2 µg/kg U7-Ctenitoxin Pn1b, 24.8 µg/kg ω-conotoxin TxVII, a, 100.6 µg/kg U6-Ctenitoxin Pnla, 33.8 µg/kg U9-Ctenitoxin Pnla, 39.8 µg/kg κ-Ctenitoxin Pn1 and 12.8 µg/kg Glacontryphan M) or Nav1.4 receptors (32 6 µg/kg µ-conotoxin CnlllC, 23 µg/kg µ-conotoxin Glllb, 36.4 µg/kg µ-Thomitoxin Hme1a, 32.5 µg/kg µ-conotoxin GvllJ, 286.6 µg/kg µ-O-conotoxin MfVIA and 39.8 µg/kg µ-Thomitoxin Hme1b) were analyzed using calculation of total Areas Under the Curve (AUC).

### DETAILED DESCRIPTION OF THE INVENTION

The nervous system sends signals by an electrical impulse that travels along the length of the nerve until it reaches a junction with another cell. Neuronal junction, also named synapse, is the site of transmission of electric nerve impulses between two nerve cells (neurons) or between a neuron and a secretory gland or muscle cell (effector). Neuronal transmission implies the release, by the presynaptic cells, of synaptic vesicles containing neurotransmitters into the synaptic space, and capture of the content of the vesicles by the postsynaptic receptors expressed by postsynaptic cells.

### Composition

In a first aspect, the present invention relates to a composition comprising at least one postsynaptic inhibitor of cholinergic neuronal transmission (PoNT) and a botulinum neurotoxin (BoNT).

By "at least one" is intended a single postsynaptic inhibitor of cholinergic neuronal transmission or a combination (i.e. a mixture) of 2, 3, 4, 5 or more postsynaptic inhibitors of cholinergic neuronal transmission.

By "postsynaptic inhibitor of cholinergic neuronal transmission (PoNT)" is meant a compound that can bind postsynaptic cell receptors and interfere with cholinergic neuronal transmission. By binding to the target receptor of neurotransmitter, ion channels or voltage-gated receptor present at the surface of postsynaptic cell, postsynaptic inhibitor of neuronal cholinergic transmission can modulate the effect of neurotransmitter (i.e. acetylcholine) release.

For the purposes of the present invention, the postsynaptic inhibitors of cholinergic neuronal transmission can be divided in two main groups: the inhibitors of peptide nature, called the postsynaptic peptides, and the inhibitor of non-peptide nature, called postsynaptic small molecules.

Thus, in the composition of the invention, the at least one postsynaptic inhibitor of cholinergic neuronal transmission is a postsynaptic peptide and/or a postsynaptic small molecule.

### Postsynaptic peptides

By "postsynaptic peptide" is meant a biologically active peptide with a specific affinity for cell surface receptor located at the postsynaptic level (typically at the membrane of the postsynaptic cell). Postsynaptic peptides used in the composition of the invention may be natural neurotoxins, or functional fragments/variants thereof, isolated from the venoms of spiders, snakes, scorpions, sea anemones, sea snails, and other animals, or synthetic peptides or derivatives thereof obtained by chemical synthesis. By "fragments or variants" of postsynaptic peptide is meant any subsequence of a postsynaptic peptide that conserves a biological activity similar to that of the parent peptide. In other word, functional fragments/variants of the postsynaptic peptide can specifically bind to and block a postsynaptic receptor expressed by a cell involved in the effector tissue identical or belonging to the same family, as that bonded and block by the native postsynaptic peptide from which the fragments or variants derived. A variant of a postsynaptic peptide may have at least 70%, at least 75%, at least 80%, at least 85%, least 90%, at least 95%, at least 96%, at least 97%, at least 98%, at least 99% sequence identity with the native postsynaptic peptide, provided that the binding and blocking activity of a target receptor is retained. Functional fragment of postsynaptic peptide includes the shortest possible peptide sequence which retains the binding and blocking activity of the native postsynaptic peptide.

By "derivative of postsynaptic peptide" is meant any modified postsynaptic peptide as compared to their native counterpart. All terminus (i.e. amidation (C-terminus), acylation (N-terminus)) and internal modifications (eg. leucine- or tyrosine-based motif) that can improve overall postsynaptic peptide stability, or its biological function are to be considered. More generally, modifications can be selected from the group comprising or consisting of phosphorylation, glycosylation, ubiquitination, nitrosylation, methylation, acetylation, lipidation, PEGylation.

In order to improve its bioavailability, the postsynaptic peptide used in the composition of the invention may comprise one or more cationic modification.

The postsynaptic peptide used in the composition of the invention may comprise at least one amino acid modification selected from an amino acid substitution, an amino acid insertion, and an amino acid deletion.

In an amino acid substitution, an amino acid residue that forms part of the postsynaptic peptide sequence is replaced with a different amino acid residue. The replacement amino acid residue may be one of the 20 standard amino acids well known in the art. The substitution may occur between amino acids considered charged amino acids (aspartic acid, glutamic acid, arginine, and lysine), uncharged, polar (asparagine, glutamine, histidine, serine, threonine, tyrosine, cysteine, methionine, tryptophan) or uncharged, hydrophobic amino acids (alanine, valine, leucine, isoleucine, phenylalanine, proline, and glycine).

Alternatively, the replacement amino acid in an amino acid substitution may be a non-standard amino acid (an amino acid that is not part of the standard set of 20 described above). By way of example, the replacement amino acid may be a basic non-standard amino acid, e.g. L-Ornithine, L-2-amino-3-guanidinopropionic acid, D-isomers of Lysine, Arginine and Ornithine, 4-hydroxyproline, 6-N-methyl lysine, 2-aminoisobutyric acid, isovaline and α-methyl serine. Methods for introducing non-standard amino acids into peptides are known in the art. A limited number of non-conservative amino acids, amino acids that are not encoded by the genetic code, and unnatural amino acids may be substituted for postsynaptic peptide amino acid residues.

In an amino acid insertion, an additional amino acid residue (one that is not normally present) is incorporated into the postsynaptic peptide amino acid sequence, thus increasing the total number of amino acid residues in said sequence.

In an amino acid deletion, an amino acid residue is removed from the postsynaptic peptide amino acid sequence, thus reducing the total number of amino acid residues in said sequence.

Methods for modifying proteins by substitution, insertion or deletion of amino acid residues are known in the art.

Depending on its size, the postsynaptic peptide comprises between 1 to 10 amino acid modifications (for example, between 1 and 9, between 1 and 8, between 1 and 7, between 1 and 6, between 1 and 5, or 4, 3, 2 or 1 amino acid modifications).

The postsynaptic peptide of the present invention can also comprise non-naturally occurring amino acid residues. Non-naturally occurring amino acids include, without limitation, trans-3-methylproline, 2,4-methano-proline, cis-4-hydroxyproline, trans-4-hydroxy-proline, N-methylglycine, allo-threonine, methyl-threonine, hydroxy-ethylcysteine, hydroxyethylhomo-cysteine, nitro-glutamine, homoglutamine, pipecolic acid, tert-leucine, norvaline, 2-azaphenylalanine, 3-azaphenyl-alanine, 4-azaphenyl-alanine, and 4-fluorophenylalanine. Several methods are known in the art for incorporating non-naturally occurring amino acid residues into proteins.

Because postsynaptic peptides of natural origin are often a mixture of several dozen neurotoxins, any conventional purification process may be useful to isolate postsynaptic peptide having the intended binding activity.

Alternatively, when highly purified postsynaptic peptide is required, synthetically produced postsynaptic peptide may be used to obtain a high level of purity.

In all embodiments, postsynaptic peptides used in the composition of the invention are peptide chain that contains approximately 110 amino acids or fewer, preferably 70 amino acids or fewer, preferably 50 amino acids or fewer, preferably 35 amino acids or fewer, and more preferably 25 amino acids or fewer. In no case can the postsynaptic peptide be less than 7 amino acids long unless it is a fragment.

When a fragment of a postsynaptic peptide is used in the composition of the invention, it can be 3, 4, 5, 6 amino acids long.

According to a more preferred embodiment, the size of the postsynaptic peptide used in the composition of the invention ranges from about 80 to 105 amino acids, preferably from about 50 to 80 amino acids, preferably from about 40 to 60 amino acids, and more preferably from about 15 to 35 amino acids.

According to a more preferred embodiment, the postsynaptic peptide used in the composition of the invention ranges contains at least one disulfide bridge.

### Small molecules

By "postsynaptic small molecule" is meant a biologically active, non-peptide, chemical molecule with a specific affinity for cell surface receptor located at the postsynaptic level (typically at the membrane of the postsynaptic cell). Postsynaptic small molecules differ from postsynaptic peptides in that they do not exhibit any peptide bond, and their molecular weight is generally inferior to 1300 g/mol. Preferably, the molecular weight of the postsynaptic small molecule used in the composition of the invention is between about 80 to 1200 g/mol, preferably between about 150 to 800 g/mol, more preferably between about 300 to 600 g/mol. These small molecules might have been extracted from the nature (plants, typically), or might be synthetic. Small molecules can generally be classified into families or related compounds; such classification will be detailed in this text when needed. It is understood that variants of small molecules within a family are to be considered.

### BoNT

By "botulinum neurotoxin" (BoNT) is meant naturally occurring eight different serotypes of botulinum neurotoxins known as type A, B, C, D, E, F,G and X as well as modified, recombinant, hybrid and chimeric botulinum neurotoxins. The expression botulinum neurotoxin and botulinum toxin are equivalent and can be used interchangeably. The term "botulinum toxin complex" or "toxin complex" as used herein refers to the approximately 150 kD botulinum toxin protein molecule (belonging to any one of botulinum toxin serotypes A-G, X), potentially along with associated endogenous non-toxin proteins (i.e., hemagglutinin protein and non-toxin non-hemagglutinin protein produced by *Clostridium botulinum* bacteria). Note, however, that the botulinum toxin complex does not need to be derived from *Clostridium botulinum* bacteria as one unitary toxin complex. For example, botulinum toxin or modified botulinum toxin may be recombinantly prepared first and then subsequently combined with the non-toxin proteins. Recombinant botulinum toxin can also be purchased (e.g., from List Biological Laboratories, Campbell, CA) and then combined with non-toxin proteins.

Mutation in the coding sequence of a BoNT that introduce one or more amino acid substitution is named modified BoNT. By "modified" botulinum neurotoxin is meant a compound that has botulinum toxin activity but contains one or more chemical or functional alterations on any part or on any amino acid chain relative to naturally occurring or recombinant native botulinum toxins. For instance, the botulinum toxin may be a modified neurotoxin that is a neurotoxin which has at least one of its amino acids deleted, modified or replaced, as compared to a native form. For instance, the botulinum toxin may be one that has been modified in a way that, for instance, enhances its properties or decreases undesirable side effects, but that still retains the desired botulinum toxin activity. The botulinum toxin may also be a portion of the overall molecule that has been shown to possess the necessary botulinum toxin activity, and in such case may be used per se or as part of a combination or conjugate molecule, for instance a fusion protein. In such case, the portion or fragment of the botulinum neurotoxin may be, for example, a 50 kDa light chain (LC) of the toxin. Alternatively, the botulinum toxin may be in the form of a botulinum toxin precursor, which may itself be non-toxic, for instance a non-toxic zinc protease that becomes toxic on proteolytic cleavage.

"Hybrid and chimeric" BoNT refers to the mixing of heavy and light chain domains of different serotypes or different subtypes of BoNT. The hybrid and chimeric within BoNT serotypes and subtypes may be natural, such as BoNT/FA and BoNT/CD, or recombinant variants of BoNT. A recombinant botulinum neurotoxin can have the light chain and/or the heavy chain thereof made recombinantly by a non-Clostridial species.

According to a preferred embodiment, the botulinum neurotoxin used in the composition of the invention is of type A, B, E or a combination of heavy and light chain of type A, B, E botulinum neurotoxin.

According to a more preferred embodiment, the botulinum neurotoxin used in the composition of the invention is of type A.

### Various effector tissues

In all embodiments, the selection of the at least one postsynaptic inhibitor of cholinergic neuronal transmission depends on synapse type and more particularly on the postsynaptic cell involved in this synapse.

### Muscle

A synaptic connection between a motor neuron and a muscle cell is called a neuromuscular junction (NMJ). In NMJ, the postsynaptic cell can be a skeletal muscle cell, a smooth muscle cell or a cardiac muscle cell.

### Skeletal muscle cell

In one embodiment, when the postsynaptic cell is a skeletal muscle cell, the at least one postsynaptic inhibitor of cholinergic neuronal transmission used in the composition according to the invention binds to at least one receptor expressed by a skeletal muscle cell, said receptor being selected from the group comprising or consisting of nicotinic acetylcholine receptors (nAChRs), and more preferably (α1)₂β1δε or (α1)₂β1δy nAChRs, ryanodine receptors type 1 (RYR1), voltage-dependent L type calcium channel CaV 1.1, and voltage-gated sodium channel Nav1.4.

When two or more postsynaptic inhibitors of cholinergic neuronal transmission (PoNT) are used as ingredients in the composition according to the invention, the receptors expressed by the skeletal cell and targeted by these PoNT may belong to the same receptor family or to several distinct families of receptors.

### Small molecule (skeletal)

In this embodiment, when the at least one synaptic inhibitor of neuronal transmission is a postsynaptic small molecule, it is selected according to the target postsynaptic receptor expressed by a skeletal muscle cell as listed in Table 1.

**Table 1: postsynaptic small molecules and skeletal muscle cell as postsynaptic cell**

| **Target postsynaptic receptor** | **Name of postsynaptic small molecule** | **MW (g/mol)** |
|---|---|---|
| nAChr (α1)₂β1δε or (α1)₂β1δγ | Rapacuronium | 597.9 |
| | Mivacurium | 1029.3 |
| | Atracurium | 929.2 |
| | Doxacurium | 1106.1 |
| | Cisatracurium | 1243.5 |
| | Vecuronium | 557.8 |
| | Rocuronium | 529.8 |
| | Pancuronium | 572.9 |
| | D-Tubocurarine | 609.7 |
| | Pipecuronium | 602.9 |
| | Suxamethonium | 290.4 |
| | Decamethonium | 258.5 |
| RYR1 | Dantrolene | 314.2 |
| | Azumolene | 349.1 |
| CaV 1.1 | Dihydropyridine | 81.1 |
| | Amlodipine | 408.9 |
| | Aranidipine | 388.4 |
| | Azelnidipine | 582.6 |
| | Barnidipine | 491.5 |
| | Benidipine | 505.5 |
| | Cilnidipine | 492.5 |
| | Clevidipine | 456.3 |
| | Efonidipine | 631.7 |
| | Elgodipine | 524.5 |
| | Felodipine | 384.3 |
| | Flordipine | 510.5 |
| | Iganidipine | 526.6 |
| | Isradipine | 371.4 |
| | Lacidipine | 455.5 |
| | Lercanidipine | 611.7 |
| | Levamlodipine | 408.9 |
| | Manidipine | 610.7 |
| | Nicardipine | 479.5 |
| | Nifedipine | 346.3 |
| | Nilvadipine | 385.4 |
| | Nimodipine | 418.4 |
| | Nisoldipine | 388.4 |
| | Nitrendipine | 360.4 |
| | Pranidipine | 448.5 |
| | Riodipine | 367.4 |
| | Verapamil | 454.6 |
| | Gallopamil | 484.6 |
| | Dimeditiapramine | 555.7 |
| | Diltiazem | 414.5 |

Nicotinic acetylcholine receptors, being a member of ligand-gated cationic channels, mediate fast synaptic transmission. In mammals, there are 16 different nAChR subunits: nine different α-subunits (α1-7, α9 and α10), four β-subunits (β1-4), as well as γ, δ and ε subunits. Five of these subunits combine to form muscle nAChR subtypes (α1β1γδ and α1β1δε) which are found at neuromuscular junctions.

In this embodiment, when the at least one synaptic inhibitor of neuronal transmission is a postsynaptic small molecule that binds (α1)₂β1δε or (α1)₂β1δy nAChRs, it is selected from the group comprising or consisting of rapacuronium, mivacurium, atracurium, doxacurium, cisatracurium, vecuronium, rocuronium, pancuronium, D-tubocurarine, pipecuronium, suxamethonium, decamethonium, or a combination thereof.

Any other small molecule that binds (α1)₂β1δε or (α1)₂β1δy nAChRs known in the art may be used as one synaptic inhibitor of neuronal transmission in the composition of the invention.

In a more preferred embodiment, when the target receptor expressed by a skeletal muscle cell is (α1)₂β1δε or (α1)₂β1δy nAChRs, the at least one postsynaptic small molecule used in the composition of the invention is pancuronium.

Ryanodine receptor isoform-1 (RyR1) is a major calcium channel in skeletal muscle important for excitation-contraction coupling. It is a six transmembrane homotetrameric protein located in the sarcoplasmic reticulum (SR) and functions to release Ca²⁺ from the SR to produce skeletal muscle contraction.

In this embodiment, when the at least one synaptic inhibitor of neuronal transmission is a postsynaptic small molecule that binds RyR1, it is selected form the group comprising or consisting of dantrolene, azumolene, or a combination thereof.

In a more preferred embodiment, when the target receptor expressed by a skeletal muscle cell is RyR1, the at least one postsynaptic small molecule used in the composition of the invention is dantrolene.

Voltage-dependent, L type, calcium channel CaV 1.1 is a channel found in the transverse tubule of muscles. In skeletal muscle it associates with the ryanodine receptor RyR1 of the sarcoplasmic reticulum via a mechanical linkage. Upon skeletal muscle membrane depolarization, Cav1.1 undergoes a conformational change which allosterically activates RγR1.

In this embodiment, when the at least one synaptic inhibitor of neuronal transmission is a postsynaptic small molecule that binds CaV 1.1, it is selected from the group comprising or consisting of dihydropyridine, amlodipine, aranidipine, azelnidipine, barnidipine, benidipine, cilnidipine, clevidipine, efonidipine, elgodipine, felodipine, flordipine, iganidipine, isradipine, lacidipine, lercanidipine, levamlodipine, manidipine, nicardipine, nifedipine, nilvadipine, nimodipine, nisoldipine, nitrendipine, pranidipine, riodipine, verapamil, gallopamil, dimeditiapramine, diltiazem, or a combination thereof.

In a more preferred embodiment, when the target receptor expressed by a skeletal muscle cell is CaV 1.1, the at least one postsynaptic small molecule used in the composition of the invention is verapamil.

### Postsynaptic peptide (skeletal)

In this embodiment, when the at least one postsynaptic inhibitor of cholinergic neuronal transmission is a postsynaptic peptide, it is selected according to the target postsynaptic receptor expressed by a skeletal muscle cell as listed in Table 2.

**Table 2: postsynaptic peptides and skeletal muscle cell as postsynaptic cell**

| **Target postsynaptic receptor** | **Name of postsynaptic peptide** | **Sequence ID** |
|---|---|---|
| nAChr (α1)₂β1δε or (α1)₂β1δγ | Azemiopsin | SEQ ID NO:1 |
| | Wagglerin-1 | SEQ ID NO:2 or 3 |
| | SYN^{®}-AKE (IUPAC name: beta-alanyl-DL-prolyl-3-aminomethyl-DL-alanine benzylamide acetic acid) | H-bAla-Pro-Dab- |
| | | NHBn.2CH₃CO₂H |
| | α-Bungarotoxin | SEQ ID NO:4 |
| | αC-Conotoxin PrXA | SEQ ID NO:5 |
| | α-cobratoxin | SEQ ID NO:6 |
| | α-Conotoxin MI | SEQ ID NO:7 or 8 |
| | α-Conotoxin GI | SEQ ID NO:7 or 9 |
| | -conotoxin PrlllE | SEQ ID NO:10 |
| | Candoxin | SEQ ID NO:11 or 12 |
| | Haditoxin | SEQ ID NO:13 or 14 |
| RYR1 | Imperacalcin | SEQ ID NO:15 or 16 |
| | Phospholipase A2 imperatoxin-1 (large subunit) | SEQ ID NO:17 or 18 |
| | Heteromtoxin (large subunit) | SEQ ID NO:17 or 19 |
| | Phospholipase A2 (large subunit) | SEQ ID NO:17 or 20 |
| | Phospholipase A2 | SEQ ID NO:17 or 21 |
| | Insecticidal Toxin LalT1 | SEQ ID NO:22 or 23 |
| | Φ-Liotoxin Lw1a | SEQ ID NO:22 or 24 |
| CaV1.1 | Calciseptine | SEQ ID NO:25 or 26 |
| | Toxin FS2 | SEQ ID NO:25 or 27 |
| | Vasotab | SEQ ID NO:28 or 29 |
| | Glacontryphan M | SEQ ID NO:30 or 31 |
| | ω-conotoxin TxVII | SEQ ID NO:32 |
| | ω-ctenitoxin Cs1a | SEQ ID NO:33 or 34 |
| | U7-ctenitoxin Pn1b | SEQ ID NO:35 |
| | U9-ctenitoxin Pnla | SEQ ID NO:36 or 37 |
| | U6-ctenitoxin Pnla | SEQ ID NO:36 or 38 |
| | κ-ctenitoxin-Pn1a | SEQ ID NO:36 or 39 |
| | ω-ctenitoxin Pr2a | SEQ ID NO:40 or 41 |
| | Toxin S4C8 | SEQ ID NO:42 or 43 |
| | Toxin C10S2C2 | SEQ ID NO:42 or 44 |
| NaV1.4 | µ-conotoxin Glllb | SEQ ID NO:45 or 46 |
| | µ-conotoxin PIIIa | SEQ ID NO:45 or 47 |
| | µ-conotoxin TIIIA | SEQ ID NO:45 or 48 |
| | µ-conotoxin GvIIJ | SEQ ID NO:49 or 50 |
| | µ-O-conotoxin MfVIA | SEQ ID NO:51 or 52 |
| | µ-conotoxin Cnlllc | SEQ ID NO:53, 54 or 95 |
| | µ-thomitoxin Hme1a | SEQ ID NO:55 or 56 |
| | µ-thomitoxin Hme1b | SEQ ID NO:55 or 57 |
| | µ-thomitoxin Hme1c | SEQ ID NO:55 or 58 |

The postsynaptic peptides able to bind nAChRs (α1β1γδ and α1β1δε) and suitable for the composition of the invention can be selected from the group comprising or consisting of azemiopsin (SEQ ID NO:1), wagglerin-1 (SEQ ID NO:2 or SEQ ID NO:3), SYN^{®}-AKE (H-bAla-Pro-Dab-NHBn.2CH₃CO₂H), α-bungarotoxin (SEQ ID NO:4), αC-conotoxin PrXA (SEQ ID NO:5), α-cobratoxin (SEQ ID NO:6), α-conotoxin MI (SEQ ID NO:7 or SEQ ID NO:8), α-conotoxin GI (SEQ ID NO:7 or SEQ ID NO:9), ψ-conotoxin-PrIIIE (SEQ ID NO:10), candoxin (SEQ ID NO:11 or SEQ ID NO:12) and haditoxin (SEQ ID NO:13 or SEQ ID NO:14), or derivatives thereof.

Consensus sequence of Wagglerin-1 (SEQ ID NO:2) has been defined relying on the sequences of the proteins having the following accession number in Uniprot database: P24335, P58930, P24335, P58930.

In a preferred embodiment, when wagglerin-1 is used in the composition of the invention, it has the sequence SEQ ID NO:3, or derivatives thereof.

Consensus sequence of α-Conotoxin MI and α-Conotoxin GI (SEQ ID NO:7) has been defined relying on the sequences of the proteins having the following accession number in Uniprot database: P01519, P01519, X5I9Y2, P01520, P0C8U4, P0DKP5, P0DKP8, P56973, P01521, P56973, P0C8U5, P0C8U4, P0C1W1, P0CAQ4, P0CAQ5, P56973, D4HPF8, D4HPE4, P0C8U5, P0C1W2, D4HPG9, D4HPF2, P0C8V1, P28878, P15471 ,P0DKP7, D4HPF6, P0DKP7, P28879.

In a preferred embodiment, when α-Conotoxin MI or α-Conotoxin GI is used in the composition of the invention, it has the sequence SEQ ID NO:8, SEQ ID NO:9, or derivatives thereof.

Consensus sequence of candoxin (SEQ ID NO:11) has been defined relying on the sequences of the proteins having the following accession number in Uniprot database: P81783, P15818, D5J9P6, D5J9P8.

In a preferred embodiment, when candoxin is used in the composition of the invention, it has the sequence SEQ ID NO:12, or a derivative thereof.

Consensus sequence of haditoxin (SEQ ID NO:13) has been defined relying on the sequences of the proteins having the following accession number in Uniprot database: A8N286, Q9W727, P82464, D5J9P3, Q7ZT13, Q800Y3, Q9YGI0.

In a preferred embodiment, when haditoxin is used in the composition of the invention, it has the sequence SEQ ID NO14, or a derivative thereof.

In an even more preferred embodiment, when the target postsynaptic receptor expressed by a skeletal muscle cell is (α1)₂β1δε or (α1)₂β1δy nAChRs, the at least one postsynaptic peptide used in the composition of the invention is α-conotoxin MI having the sequence SEQ ID NO:7, SEQ ID NO:8, or derivatives thereof.

Postsynaptic peptides able to bind RyR1 and suitable for the composition of the invention can be selected from the group comprising or consisting of imperacalcin (SEQ ID NO:15 or SEQ ID NO:16) phospholipase A2 imperatoxin-1 (large subunit) (SEQ ID NO:17 or SEQ ID NO:18), heteromtoxin (large subunit) (SEQ ID NO:17 or SEQ ID NO:19), phospholipase A2 (large subunit) (SEQ ID NO:17 or SEQ ID NO:20), phospholipase A2 (SEQ ID NO:17 or SEQ ID NO:21), insecticidal toxin LaIT1 (SEQ ID NO:22 or SEQ ID NO:23) and ϕ-liotoxin LW1a (SEQ ID NO:22 or SEQ ID NO:24), or derivatives thereof.

Consensus sequence of imperacalcin (SEQ ID NO:15) has been defined relying on the sequences of the proteins having the following accession number in Uniprot database: P59868, P60252, P60253, A0A1L4BJ42, P60254, P0DPT1, B8QG00, P0DM30, A0A1B31J19, L0GBR1, A0A1B31J24, A0A1W7RAU1, A0A224X3X5, A0A224X3Z6, A0A1V1WBN6.

In a preferred embodiment, when imperacalcin is used in the composition of the invention, it has the sequence SEQ ID NO:16, or a derivative thereof.

Consensus sequence of phospholipase A2 imperatoxin-1 (large subunit), heteromtoxin (large subunit), phospholipase A2 (large subunit), and phospholipase A2 (SEQ ID NO:17) has been defined relying on the sequences of the proteins having the following accession number in Uniprot database: Q6T178, Q3YAU5, P59888, H2CYP4, P0DMI6, A0A1L4BJ57, A0A1W7R9Y9, P0C8L9, A0A1W7RA04, A0A1L4BJ64.

In a preferred embodiment, when phospholipase A2 imperatoxin-1 (large subunit), or heteromtoxin (large subunit), or phospholipase A2 (large subunit), or phospholipase A2 is used in the composition of the invention, it has the sequence SEQ ID NO:18, SEQ ID NO:19, SEQ ID NO:20, SEQ ID NO:21, or a derivative thereof.

Consensus sequence of insecticidal toxin LaIT1 and ϕ-liotoxin LW1a (SEQ ID NO:22) has been defined relying on the sequences of the proteins having the following accession number in Uniprot database: P0C5F2, P0DJ08.

In a preferred embodiment, when insecticidal Toxin LaIT1 or ϕ-liotoxin LW1a is used in the composition of the invention, it has the sequence SEQ ID NO:23, SEQ ID NO:24, or a derivative thereof.

In a even more preferred embodiment, when the target receptor expressed by a skeletal muscle cell is RyR1, the at least one postsynaptic peptide used in the composition of the invention is insecticidal toxin LaIT1 having the sequence SEQ ID NO:22 or SEQ ID NO:23, or derivatives thereof.

Postsynaptic peptides able to bind CaV 1.1 and suitable for the composition of the invention can be selected from the group comprising or consisting of calciseptine (SEQ ID NO:25 or SEQ ID NO:26), toxin FS2 (SEQ ID NO:25 or SEQ ID NO:27), vasotab (SEQ ID NO:28 or SEQ ID NO:29), glacontryphan M (SEQ ID NO:30 or SEQ ID NO:31), ω-conotoxin-TxVll (SEQ ID NO:32), ω-ctenitoxin-Csla (SEQ ID NO:33 or SEQ ID NO:34), U7-ctenitoxin-Pn1b (SEQ ID NO:35), U9-ctenitoxin-Pn1a (SEQ ID NO:36 or SEQ ID NO:37), U6-ctenitoxin-Pnla (SEQ ID NO:36 or SEQ ID NO:38), κ-ctenitoxin-Pn1a (SEQ ID NO:36 or SEQ ID NO:39), ω-ctenitoxin-Pr2a (SEQ ID NO:40 or SEQ ID NO:41), toxin S4C8 (SEQ ID NO:42 or SEQ ID NO:43) and toxin C10S2C2 (SEQ ID NO:42 or SEQ ID NO:44), or derivatives thereof.

Consensus sequence of calciseptine and toxin FS2 (SEQ ID NO:25) has been defined relying on the sequences of the proteins having the following accession number in Uniprot database: P22947, P01414, P25684, P25683.

In a preferred embodiment, when calciseptine or toxin FS2 is used in the composition of the invention, it has the sequence SEQ ID NO: 26, SEQ ID NO:27, or a derivative thereof.

Consensus sequence of vasotab (SEQ ID NO:28) has been defined relying on the sequences of the proteins having the following accession number in Uniprot database: P84843, C1IBZ2, A0A0K8TPQ2, C8YJB4, C8YJB3.

In a preferred embodiment, when vasotab is used in the composition of the invention, it has the sequence SEQ ID NO:29, or a derivative thereof.

Consensus sequence of glacontryphan M (SEQ ID NO:30) has been defined relying on the sequences of the proteins having the following accession number in Uniprot database: U6C1Y3, A0A0K8TU78, A0A1P8NVU1, F5C3T9, A0A1P8NVS9, A0A1P8NVU0.

In a preferred embodiment, when glacontryphan M is used in the composition of the invention, it has the sequence SEQ ID NO:31, or a derivative thereof.

Consensus sequence of ω-ctenitoxin-Cs1a (SEQ ID NO:33) has been defined relying on the sequences of the proteins having the following accession number in Uniprot database: P81694, B6DCP3, B6DCP1, B6DCP4, B6DCN7.

In a preferred embodiment, when ω-ctenitoxin-Cs1a is used in the composition of the invention, it has the sequence SEQ ID NO:34, or a derivative thereof.

Consensus sequence of U9-ctenitoxin-Pn1a, U6-ctenitoxin-Pn1a, and κ-ctenitoxin-Pn1a (SEQ ID NO:36) has been defined relying on the sequences of the proteins having the following accession number in Uniprot database: O76200, 076201, 076201, P0C2S6, P84000, P83895, P30288, P37045, P81793.

In a preferred embodiment, when U9-ctenitoxin-Pn1a, or U6-ctenitoxin-Pn1a, or κ-ctenitoxin-Pnla is used in the composition of the invention, it has the sequence SEQ ID NO:37, SEQ ID NO: 38, SEQ ID NO:39, or a derivative thereof.

Consensus sequence of ω-ctenitoxin-Pr2a (SEQ ID NO:40) has been defined relying on the sequences of the proteins having the following accession number in Uniprot database: P81792, P83902, P0C2S7, P84011, P83901.

In a preferred embodiment, when ω-ctenitoxin-Pr2a is used in the composition of the invention, it has the sequence SEQ ID NO:41, or a derivative thereof.

Consensus sequence of toxin S4C8 and toxin C10S2C2 (SEQ ID NO:42) has been defined relying on the sequences of the proteins having the following accession number in Uniprot database: P22947, P01414, P25684, P25683.

In a preferred embodiment, when toxin S4C8 or toxin C10S2C2 is used in the composition of the invention, it has the sequence SEQ ID NO:43, SEQ ID NO:44, or a derivative thereof.

In an even more preferred embodiment, when the target receptor expressed by a skeletal muscle cell is CaV 1.1, the at least one postsynaptic peptide used in the composition of the invention is U7-ctenitoxin-Pn1b having the sequence SEQ ID NO: 35 or calciseptine having the sequence SEQ ID NO:25 or SEQ ID NO:26, or derivatives thereof.

Nav 1.4 channel is primarily found in the sarcolemma and T-tubule membranes of skeletal muscle fibers. A depolarizing current through the channel pore initiates the skeletal muscle action potential and leads to contraction.

Mu-conotoxins (µ-conotoxins) directly abolish muscle action potential by binding to the Nav 1.4 channels. Mu-conopeptides are isolated from the venoms of marine cone snails of the genus *Conus.* The primary structure of mu-conopeptides is organized with 15-30 amino acids folded by disulfide bridges. The µ-conotoxins have a characteristic CC-Xm-C-Xn-C-Xp-CC framework, where the six cysteines can yield three possible disulfide connectivities (i.e., Cysl-CyslV, Cysll-CysV, Cyslll-CysVl). The number of residues included within the three loops (m,n,p) of µ-conotoxins is the basis for the division into several structural subgroups (m may vary from 5 to 9 amino acid residues, n may vary from 3 to 4 amino acid residues and p may vary from 3 to 5 amino acid residues).

In some embodiments, postsynaptic peptides able to bind NaV 1.4 channels and suitable for the composition of the invention can be selected from the group comprising or consisting of µ-conotoxin Glllb (SEQ ID NO:45 or SEQ ID NO:46), µ-conotoxin Tllla (SEQ ID NO:45 or SEQ ID NO:48), µ-conotoxin PIIIa (SEQ ID NO:45 or SEQ ID NO:47), µ-conotoxin GvllJ (SEQ ID NO:49 or SEQ ID NO:50), µ-O-conotoxin MfVIA (SEQ ID NO:51 or SEQ ID NO:52), µ-conotoxin Cnlllc (SEQ ID NO:53, SEQ ID NO:54 or SEQ ID NO:95), µ-thomitoxin-Hme1a (SEQ ID NO:55 or SEQ ID NO:56), µ-thomitoxin-Hme1b (SEQ ID NO:55 or SEQ ID NO:57), µ-thomitoxin-Hme1c (SEQ ID NO:55 or SEQ ID NO:58), or derivatives thereof.

Consensus sequence of µ-conotoxin Glllb, µ-conotoxin TIIIA, and µ-conotoxin PIIIa (SEQ ID NO:45) has been defined relying on the sequences of the proteins having the following accession number in Uniprot database: P01523, P01524, P05482, P0C349, P0DKQ9, P0DKQ9, X5IGY9, A0A0K8TUB1, P0CH16, P58925.

In a preferred embodiment, when µ-conotoxin Glllb or µ-conotoxin TIIIA or when µ-conotoxin PIIIa is used in the composition of the invention, it has the sequence SEQ ID NO:46, SEQ ID NO:48, SEQ ID NO:47, or derivatives thereof.

Consensus sequence of µ-conotoxin GvIIJ (SEQ ID NO:49) has been defined relying on the sequences of the proteins having the following accession number in Uniprot database: X5IWS1, X5IA08, Q71KS8.

In a preferred embodiment, when µ-conotoxin GvllJ is used in the composition of the invention, it has the sequence SEQ ID NO:50, or a derivative thereof.

Consensus sequence of µ-conotoxin Cnlllc (SEQ ID NO:53) has been defined relying on the sequences of the proteins having the following accession number in Uniprot database: C1J5M5, C1J5M6, C1J5M7, I1SB07, P0CE77, Q86DU6, P60207, P0C1T9, P0C1U2, P0C195, P0C1U1, P0C1U0, P0CY74.

In a preferred embodiment, the postsynaptic peptide used in the composition of the invention is µ-conotoxin Cnlllc or a variant thereof.

In a more preferred embodiment, µ-conotoxin Cnlllc is used in the composition of the invention, it has the sequence SEQ ID NO: 54 or SEQ ID NO:95, or a derivative thereof.

Consensus sequence of µ-O-conotoxin MfVIA (SEQ ID NO:51) has been defined relying on the sequences of the proteins having the following accession number in Uniprot database: P0DM15, A0A0K2S5L8, A0A0K2S5L9, A0A0K2S5N5, A0A0K2S6J2, A0A0K2S5L0, A0A0K2S5M2, A0A0K2S6H9, A0A0K2S5M9, A0A0K2S5M7, U6C1V9, P56708, U6C2E4, W4VSL3.

In a preferred embodiment, when µ-O-conotoxin MfVIA is used in the composition of the invention, it has the sequence SEQ ID NO:52, or a derivative thereof.

Consensus sequence of µ-thomitoxin Hme1a, µ-thomitoxin Hme1b, and µ-thomitoxin Hme1c (SEQ ID NO:55) has been defined relying on the sequences of the proteins having the following accession number in Uniprot database: P85505, P85506, C0HJK5.

In a preferred embodiment, when µ-thomitoxin-Hmela, µ-thomitoxin-Hme1b or µ-thomitoxin-Hme1c is used in the composition of the invention, it has the sequence SEQ ID NO:56, SEQ ID NO:57, SEQ ID NO:58, or a derivative thereof.

### Smooth muscle

In another embodiment, when the postsynaptic cell is a smooth muscle cell, the at least one postsynaptic inhibitor of cholinergic neuronal transmission used in the composition according to the invention binds to at least one receptor expressed by a smooth muscle cell, said receptor being selected from the group comprising or consisting of muscarinic acetylcholine receptors (mAChRs), and more preferably M3 subtype mAChRs, ryanodine receptors type 2 (RYR2), voltage-dependent L type calcium channel CaV 1.2, and voltage-gated sodium channel Nav1.5.

When two or more postsynaptic inhibitors of cholinergic neuronal transmission (PoNT) are used as ingredients in the composition according to the invention, the receptors expressed by the smooth muscle cell and targeted by these PoNT may belong to the same receptor family or to several distinct families of receptors.

### Small molecule (smooth)

In this embodiment, when the at least one synaptic inhibitor of neuronal transmission is a postsynaptic small molecule, it is selected according to the target postsynaptic receptor expressed by a smooth muscle cell as listed in Table 3.

**Table 3: postsynaptic small molecules and smooth muscle cell as postsynaptic cell**

| **Target postsynaptic receptor** | **Name of postsynaptic small molecule** | **MW (g/mol)** |
|---|---|---|
| mAChRs M3 | Atropine | 289.4 |
| | Scopolamine | 303.3 |
| | Hyoscyamine | 289.4 |
| | Ipratropium | 426.5 |
| | Tropicamide | 284.4 |
| | Flavoxate | 391.5 |
| | Oxybutynin | 394 |
| | Tiotropium | 392.5 |
| | Cyclopentolate | 291.4 |
| | Tolterodine | 325.5 |
| | Procyclidine | 287.4 |
| | Aclidinium | 564.6 |
| | Solifenacin | 480.5 |
| | Darifenacin | 426.6 |
| | 4-DAMP | 451.4 |
| | DAU-5884 | 351.8 |
| | J-104129 | 500.6 |
| | Zamifenacin | 531.6 |
| RYR2 | JTV519 | 540.7 |
| CaV 1.2 | Dihydropyridine | 81.1 |
| | Amlodipine | 408.9 |
| | Aranidipine | 388.4 |
| | Azelnidipine | 582.6 |
| | Barnidipine | 491.5 |
| | Benidipine | 505.5 |
| | Cilnidipine | 492.5 |
| | Clevidipine | 456.3 |
| | Efonidipine | 631.7 |
| | Elgodipine | 524.5 |
| | Felodipine | 384.3 |
| | Flordipine | 510.5 |
| | Iganidipine | 526.6 |
| | Isradipine | 371.4 |
| | Lacidipine | 455.5 |
| | Lercanidipine | 611.7 |
| | Levamlodipine | 408.9 |
| | Manidipine | 610.7 |
| | Nicardipine | 479.5 |
| | Nifedipine | 346.3 |
| | Nilvadipine | 385.4 |
| | Nimodipine | 418.4 |
| | Nisoldipine | 388.4 |
| | Nitrendipine | 360.4 |
| | Pranidipine | 448.5 |
| | Riodipine | 367.4 |
| | Verapamil | 454.6 |
| | Gallopamil | 484.6 |
| | Dimeditiapramine | 555.7 |
| | Diltiazem | 414.5 |

Muscarinic acetylcholine receptors are an important subfamily of class A, G protein-coupled receptors (GPCRs). There are five mAChR subtypes (M1-M5), which differ in their expression pattern, physiological function, and G protein coupling. Three subtypes (M1, M3, and M5) signal predominantly through activation of G proteins from the Gq/11 family, whereas M2 and M4 mAChRs primarily signal through the Gi/o family of G proteins. M3 mAChRs are present in smooth muscle cells. Furthermore, the muscarinic M3 receptor is considered to be the principal receptor subtype mediating the parasympathetic contractile response in smooth muscle tissues.

In this embodiment, when the at least one synaptic inhibitor of neuronal transmission is a postsynaptic small molecule that binds mAChRs M3, it is selected from the group comprising or consisting of atropine, scopolamine, hyoscyamine, ipratropium, tropicamide, flavoxate, oxybutynin, tiotropium, cyclopentolate, tolterodine, procyclidine, aclidinium, solifenacin, darifenacin, 4-DAMP, DAU-5884, J-104129, zamifenacin, or a combination thereof.

In a more preferred embodiment, when the target receptor expressed by a smooth muscle cell is mAChRs M3, the at least one postsynaptic small molecule used in the composition of the invention is darifenacin.

Ryanodine receptor isoform-2 (RyR2) is one of the three major calcium channels in smooth muscle important for excitation-contraction coupling. The contribution of RyR2 to smooth muscle calcium spark generation is well illustrated.

In a more preferred embodiment, when the target receptor expressed by a smooth muscle cell is RyR2, the at least one postsynaptic small molecule used in the composition of the invention is JTV519.

Voltage-dependent, L type, calcium channel CaV 1.2 is a calcium channel found in the membrane of smooth muscle cells. CaV1.2 is an integral cell membrane protein complex that mediates the influx of Ca2⁺ into the cell in response to membrane depolarization, thereby inducing muscle contraction. In smooth muscle cells it associates with the ryanodine receptor RyR2 of the sarcoplasmic reticulum via a mechanical linkage.

In this embodiment, when the at least one synaptic inhibitor of neuronal transmission is a postsynaptic small molecule that binds CaV 1.2, it is selected from the group comprising or consisting of dihydropyridine, amlodipine, aranidipine, azelnidipine, barnidipine, benidipine, cilnidipine, clevidipine, efonidipine, elgodipine, felodipine, flordipine, iganidipine, isradipine, lacidipine, lercanidipine, levamlodipine, manidipine, nicardipine, nifedipine, nilvadipine, nimodipine, nisoldipine, nitrendipine, pranidipine, riodipine, verapamil, gallopamil, dimeditiapramine, diltiazem, or a combination thereof.

In a more preferred embodiment, when the target receptor expressed by a smooth muscle cell is CaV 1.2, the at least one postsynaptic small molecule used in the composition of the invention is verapamil.

### Post-synaptic peptide (smooth)

In this embodiment, when the at least one synaptic inhibitor of neuronal transmission is a postsynaptic peptide, it is selected according to the target postsynaptic receptor expressed by a smooth muscle cell as listed in Table 4.

**Table 4: postsynaptic peptides and smooth muscle cell as postsynaptic cell**

| **Target postsynaptic receptor** | **Name of postsynaptic peptide** | **Sequence ID** |
|---|---|---|
| mAChR M3 | Muscarinic toxin alpha | SEQ ID NO: 59 or 60 |
| RYR2 | Imperacalcin | SEQ ID NO:15 or 16 |
| | Phospholipase A2 imperatoxin-1 (large subunit) | SEQ ID NO:17 or 18 |
| | Heteromtoxin (large subunit) | SEQ ID NO:17 or 19 |
| | Phospholipase A2 (large subunit) | SEQ ID NO:17 or 20 |
| | Phospholipase A2 | SEQ ID NO:17 or 21 |
| | Insecticidal Toxin LalT1 | SEQ ID NO:22 or 23 |
| | ϕ-Liotoxin-Lw1a | SEQ ID NO:22 or 24 |
| CaV1.2 | Calciseptine | SEQ ID NO:25 or 26 |
| | Toxin FS2 | SEQ ID NO:25 or 27 |
| | Vasotab | SEQ ID NO:28 or 29 |
| | Glacontryphan M | SEQ ID NO:30 or 31 |
| | ω-conotoxin-TxVII | SEQ ID NO:32 |
| | ω-ctenitoxin-Cs1a | SEQ ID NO:33 or 34 |
| | U7-ctenitoxin-Pn1b | SEQ ID NO:35 |
| | U9-ctenitoxin-Pn1a | SEQ ID NO:36 or 37 |
| | U6-ctenitoxin-Pn1a | SEQ ID NO:36 or 38 |
| | κ-ctenitoxin-Pn1a | SEQ ID NO:36 or 39 |
| | ω-ctenitoxin-Pr2a | SEQ ID NO:40 or 41 |
| | Toxin S4C8 | SEQ ID NO:42 or 43 |
| | Toxin C10S2C2 | SEQ ID NO:42 or 44 |
| | ω-theraphotoxin-Cc1a | SEQ ID NO:61 |
| NaV1.5 | β-mammal toxin Css2 | SEQ ID NO:62 or 63 |
| | α-like toxin BmK-M1 | SEQ ID NO:64 or 65 |
| | µ-thomitoxin-Hme1a | SEQ ID NO:55 or 56 |
| | µ-thomitoxin-Hme1b | SEQ ID NO:55 or 57 |
| | µ-thomitoxin-Hme1c | SEQ ID NO:55 or 58 |
| | β/κ-theraphotoxin-Cg1a | SEQ ID NO:66 or 67 |
| | κ-theraphotoxin-Cg1a | SEQ ID NO:68 or 69 |
| | δ-theraphotoxin-Cg1a 1 | SEQ ID NO:70 or 71 |
| | δ-theraphotoxin-Cg1a 2 | SEQ ID NO:70 or 72 |
| | δ-theraphotoxin-Cg1a 3 | SEQ ID NO:70 or 73 |

Consensus sequence of muscarinic toxin α (SEQ ID NO:59) has been defined relying on the sequences of the proteins having the following accession number in Uniprot database: P80494, Q9PSN1, P81030, P86419, P80495, P85092, P25518, P17696, P82463, P60234, P18328, Q8QGR0, P81031.

Postsynaptic peptides able to bind mAChRs M3 and suitable for the composition of the invention are muscarinic toxin α (SEQ ID NO: 59 or SEQ ID NO:60), or a derivative thereof.

Postsynaptic peptides able to bind RyR2 and suitable for the composition of the invention can be selected from the group comprising or consisting of imperacalcin (SEQ ID NO:15 or 16), phospholipase A2 imperatoxin-1 (large subunit) (SEQ ID NO:17 or SEQ ID NO:18), heteromtoxin (large subunit) (SEQ ID NO:17 or SEQ ID NO:19), phospholipase A2 (large subunit) (SEQ ID NO:17 or SEQ ID NO:20), phospholipase A2 (SEQ ID NO:17 or SEQ ID NO:21), insecticidal toxin LaIT1 (SEQ ID NO:22 or SEQ ID NO:23) and Phi-liotoxin LW1a (SEQ ID NO:22 or SEQ ID NO:24), or derivatives thereof.

In a preferred embodiment, when imperacalcin is used in the composition of the invention, it has the sequence SEQ ID NO:16, or a derivative thereof.

In a preferred embodiment, when phospholipase A2 imperatoxin-1 (large subunit), or heteromtoxin (large subunit), or phospholipase A2 (large subunit), or phospholipase A2 is used in the composition of the invention, it has the sequence SEQ ID NO:18, SEQ ID NO:19, SEQ ID NO:20, SEQ ID NO:21, or a derivative thereof.

In a preferred embodiment, when Insecticidal Toxin LalT1 or ϕ-liotoxin LW1a is used in the composition of the invention, it has the sequence SEQ ID NO:23, SEQ ID NO:24, or a derivative thereof.

In an even more preferred embodiment, when the target postsynaptic receptor expressed by a smooth muscle cell is RyR2, the at least one postsynaptic peptide used in the composition of the invention is Insecticidal Toxin LalT1 having the sequence SEQ ID NO:22, SEQ ID NO:23, or derivatives thereof.

Postsynaptic peptides able to bind CaV 1.2 and suitable for the composition of the invention can be selected from the group comprising or consisting of calciseptine (SEQ ID NO:25 or SEQ ID NO:26), toxin FS2 (SEQ ID NO:25 or SEQ ID NO:27), vasotab (SEQ ID NO:28 or SEQ ID NO:29), glacontryphan M (SEQ ID NO:30 or SEQ ID NO:31), ω-conotoxin-TxVll (SEQ ID NO:32), ω-ctenitoxin-Cs1a (SEQ ID NO:33 or SEq ID NO:34), U7-ctenitoxin-Pn1b (SEQ ID NO:35), U9-ctenitoxin-Pn1a (SEQ ID NO:36 or SEQ ID NO:37), U6-ctenitoxin-Pnla (SEQ ID NO:36 or SEQ ID NO:38), κ-ctenitoxin-Pn1a (SEQ ID NO:36 or SEQ ID NO:39), ω-ctenitoxin-Pr2a (SEQ ID NO:40 or SEQ ID NO:41), toxin S4C8 (SEQ ID NO:42 or SEQ ID NO:43), toxin C10S2C2 (SEQ ID NO:42 or SEQ ID NO:44) and ω-theraphotoxin-Cc1a (SEQ ID NO:61), or derivatives thereof.

In a preferred embodiment, when calciseptine or toxin FS2 is used in the composition of the invention, it has the sequence SEQ ID NO:26, SEQ ID NO:27, or a derivative thereof.

In a preferred embodiment, when vasotab is used in the composition of the invention, it has the sequence SEQ ID NO:29, or a derivative thereof.

In a preferred embodiment, when glacontryphan M is used in the composition of the invention, it has the sequence SEQ ID NO: 31, or a derivative thereof.

In a preferred embodiment, when ω-ctenitoxin-Cs1a is used in the composition of the invention, it has the sequence SEQ ID NO:34, or a derivative thereof.

In a preferred embodiment, when U9-ctenitoxin-Pn1a, or U6-ctenitoxin-Pn1a, or κ-ctenitoxin-Pnla is used in the composition of the invention, it has the sequence SEQ ID NO:37, SEQ ID NO:38, SEQ ID NO:39, or a derivative thereof.

In a preferred embodiment, when ω-ctenitoxin-Pr2a is used in the composition of the invention, it has the sequence SEQ ID NO:41, or a derivative thereof.

In a preferred embodiment, when toxin S4C8 or toxin C10S2C2 is used in the composition of the invention, it has the sequence SEQ ID NO:43, SEQ ID NO:44, or a derivative thereof.

In an even more preferred embodiment, when the target postsynaptic receptor expressed by a smooth muscle cell is CaV1.2, the at least one postsynaptic peptide used in the composition of the invention is U7-ctenitoxin-Pn1b having the sequence SEQ ID NO:35 or calciseptine having the sequence SEQ ID NO:25 or SEQ ID NO:26, or derivatives thereof.

When more specificity towards the target postsynaptic receptor CaV1.2 is required, the at least one postsynaptic peptide used in the composition of the invention is ω-theraphotoxin-Cc1a (SEQ ID NO:61), or a derivative thereof.

NaV1.5 (encoded by SCN5A gene), is expressed in human smooth muscle cells and the interstitial cells of Cajal (ICC) of the small intestine and colon. Among other functions, ICC are gastrointestinal pacemakers that generate cyclic depolarizations (slow waves) transmitted to the smooth muscle to provide the electrical stimulus for contraction. In human gastrointestinal smooth muscle, NaV channels appear to be excitatory for slow waves and NaV1.5 is functionally relevant in the human GI tract, as pharmacologic block of NaV1.5 is associated with constipation.

Postsynaptic peptides able to bind NaV1.5 and suitable for the composition of the invention can be selected from the group comprising or consisting of β-mammal toxin Css2 (SEQ ID NO:62 or SEQ ID NO:63), α-like toxin BmK-M1 (SEQ ID NO:64 or SEQ ID NO:65), µ-thomitoxin-Hme1a (SEQ ID NO:55 or SEQ ID NO:56), µ-thomitoxin-Hme1b (SEQ ID NO:55 or SEQ ID NO:57), µ-thomitoxin-Hme1c (SEQ ID NO:55 or SEQ ID NO:58), β/κ-theraphotoxin-Cg1a (SEQ ID NO:66 or SEQ ID NO:67), κ-theraphotoxin-Cg1a (SEQ ID NO:68 or SEQ ID NO:69), δ-theraphotoxin-Cg1a 1 (SEQ ID NO:70 or SEQ ID NO:71), δ-theraphotoxin-Cg1a 2 (SEQ ID NO:70 or SEQ ID NO:72) and δ-theraphotoxin-Cg1a 3 (SEQ ID NO:70 or SEQ ID NO:73), or derivatives thereof.

Consensus sequence of β-mammal toxin Css2 (SEQ ID NO:62) has been defined relying on the sequences of the proteins having the following accession number in Uniprot database: P08900, P01495, P56646, P60267, P59897, P18926, P59898, P60266, P0DL83, P0CH41, P45662, P45666, Q95WD2, P80076, Q7Z1K9, Q7Z1K8, Q9TWL0, C0HK69.

In a preferred embodiment, when β-mammal toxin Css2 is used in the composition of the invention, it has the sequence SEQ ID NO:63, or a derivative thereof.

Consensus sequence of α-like toxin BmK-M1 (SEQ ID NO:64) has been defined relying on the sequences of the proteins having the following accession number in Uniprot database: Q9GQV6, P59360, P58488, Q6IZE0, P59354, P01488, G4WFQ2, P01487, P17728, B8XGY6, Q9N682, P01483, P09981, B6A8S0, B6A8R9, P58328, P15227, P45698, Q9NJC8, P86405, P59896, E7D081, E7D082, P55902, D8UWD5, D8UWD4, , P0DJH8, D8UWD6, P60257, 061705, Q9NJC5, P60255, P01489, P84646, B8XGX9, P83644, P84614.

In a preferred embodiment, when α-like toxin BmK-M1 is used in the composition of the invention, it has the sequence SEQ ID NO:65, or a derivative thereof.

Consensus sequence of β/κ-theraphotoxin-Cg1a (SEQ ID NO:66) has been defined relying on the sequences of the proteins having the following accession number in Uniprot database: P62520, P84836, P84835, B1P1C8, B1P1D0, B1P1C9, P0CH52, P60980.

In a preferred embodiment, when β/κ-theraphotoxin-Cg1a is used in the composition of the invention, it has the sequence SEQ ID NO:67, or a derivative thereof.

Consensus sequence of κ-theraphotoxin-Cg1a (SEQ ID NO:68) has been defined relying on the sequences of the proteins having the following accession number in Uniprot database: P0C247, B1P1E4, B1P1H1, B1P1A0.

In a preferred embodiment, when κ-theraphotoxin-Cg1a is used in the composition of the invention, it has the sequence SEQ ID NO:69, or a derivative thereof.

Consensus sequence of δ-theraphotoxin-Cg1a 1, δ-theraphotoxin-Cg1a 2 and δ-theraphotoxin-Cg1a 3 (SEQ ID NO:70) has been defined relying on the sequences of the proteins having the following accession number in Uniprot database: B1P1B7, B1P1B8, ADA482ZD06, B1P1B9.

In a preferred embodiment, when δ-theraphotoxin-Cg1a 1, or δ-theraphotoxin-Cg1a 2, or δ-theraphotoxin-Cg1a 3 is used in the composition of the invention, it has the sequence SEQ ID NO:71, SEQ ID NO:72, SEQ ID NO:73, or a derivative thereof.

In a preferred embodiment, when µ-thomitoxin-Hme1a or µ-thomitoxin-Hme1b or µ-thomitoxin-Hme1c is used in the composition of the invention, it has the sequence SEQ ID NO:56, SEQ ID NO:57, SEQ ID NO:58, or a derivative thereof.

In an even more preferred embodiment, when the target postsynaptic receptor expressed by a smooth muscle cell is Nav1.5, the at least one postsynaptic peptide used in the composition of the invention is κ-theraphotoxin-Cg1a having the sequence 68 or SEQ ID NO:69, or β-mammal toxin Css2 having the sequence SEQ ID NO:62 or SEQ ID NO:63, or derivatives thereof.

### Cardiac muscle

In another embodiment, when the postsynaptic cell is a cardiac muscle cell, the at least one postsynaptic inhibitor of cholinergic neuronal transmission used in the composition according to the invention binds to at least one receptor expressed by a cardiac muscle cell, said receptor being selected from the group comprising or consisting of muscarinic acetylcholine receptors (mAChRs), and more preferably M2 subtype mAChRs, ryanodine receptors type 2 (RYR2), voltage-dependent L type calcium channels CaV 1.1 or CaV 1.2, and voltage-gated sodium channel Nav1.5.

When two or more postsynaptic inhibitors of cholinergic neuronal transmission (PoNT) are used as ingredients in the composition according to the invention, the receptors expressed by the cardiac muscle cell and targeted by these PoNT may belong to the same receptor family or to several distinct families of receptors.

### Small molecule (cardiac)

In this embodiment, when the at least one synaptic inhibitor of neuronal transmission is a postsynaptic small molecule, it is selected according to the target postsynaptic receptor expressed by a cardiac muscle cell as listed in Table 5.

**Table 5: postsynaptic small molecules and cardiac muscle cell as postsynaptic cell**

| **Target postsynaptic receptor** | **Name of postsynaptic small molecule** | **MW (g/mol)** |
|---|---|---|
| mAChRs M2 | Atropine | 289.4 |
| | Scopolamine | 303.3 |
| | Hyoscyamine | 289.4 |
| | Ipratropium | 426.5 |
| | Tropicamide | 284.4 |
| | Flavoxate | 391.5 |
| | Oxybutynin | 394 |
| | Tiotropium | 472 |
| | Cyclopentolate | 291.4 |
| | Tolterodine | 325.5 |
| | Procyclidine | 287.4 |
| | Aclidinium | 564.6 |
| | AFDX 116 | 421 |
| | AFDX-384 | 478.6 |
| | Methoctramine | 582.9 |
| | Otenzepad | 421.5 |
| | Tripitramine | 1124.4 |
| RYR2 | JTV519 | 540.7 |
| CaV 1.1 or CaV 1.2 | Dihydropyridine | 81.1 |
| | Amlodipine | 408.9 |
| | Aranidipine | 388.4 |
| | Azelnidipine | 582.6 |
| | Barnidipine | 491.5 |
| | Benidipine | 505.5 |
| | Cilnidipine | 492.5 |
| | Clevidipine | 456.3 |
| | Efonidipine | 631.7 |
| | Elgodipine | 524.5 |
| | Felodipine | 384.3 |
| | Flordipine | 510.5 |
| | Iganidipine | 526.6 |
| | Isradipine | 371.4 |
| | Lacidipine | 455.5 |
| | Lercanidipine | 611.7 |
| | Levamlodipine | 408.9 |
| | Manidipine | 610.7 |
| | Nicardipine | 479.5 |
| | Nifedipine | 346.3 |
| | Nilvadipine | 385.4 |
| | Nimodipine | 418.4 |
| | Nisoldipine | 388.4 |
| | Nitrendipine | 360.4 |
| | Pranidipine | 448.5 |
| | Riodipine | 367.4 |
| | Verapamil | 454.6 |
| | Gallopamil | 484.6 |
| | Dimeditiapramine | 555.7 |
| | Diltiazem | 414.5 |

Muscarinic acetylcholine receptors M2 are present in cardiac muscle cells. M2 muscarinic acetylcholine receptors modulate cardiac rhythm via regulation of the inward potassium current, notably.

In this embodiment, when the at least one synaptic inhibitor of neuronal transmission is a postsynaptic small molecule that binds mAChRs M2, it is selected from the group comprising or consisting of atropine, scopolamine, hyoscyamine, ipratropium, tropicamide, flavoxate, oxybutynin, tiotropium, cyclopentolate, tolterodine, procyclidine, aclidinium, AFDX 116, AFDX-384, methoctramine, otenzepad, tripitramine, or a combination thereof.

In a more preferred embodiment, when the target postsynaptic receptor expressed by a cardiac muscle cell is mAChRs M2, the at least one postsynaptic small molecule used in the composition of the invention is tolterodine.

In this embodiment, when the at least one synaptic inhibitor of neuronal transmission is a postsynaptic small molecule that binds RyR2, the at least one postsynaptic small molecule used in the composition of the invention is JTV519.

In this embodiment, when the at least one synaptic inhibitor of neuronal transmission is a postsynaptic small molecule that binds CaV 1.1 or CaV 1.2, it is selected from the group comprising or consisting of dihydropyridine, amlodipine, aranidipine, azelnidipine, barnidipine, benidipine, cilnidipine, clevidipine, efonidipine, elgodipine, felodipine, flordipine, iganidipine, isradipine, lacidipine, lercanidipine, levamlodipine, manidipine, nicardipine, nifedipine, nilvadipine, nimodipine, nisoldipine, nitrendipine, pranidipine, riodipine, verapamil, gallopamil, dimeditiapramine, diltiazem, or a combination thereof.

In a more preferred embodiment, when the target receptor expressed by a smooth muscle cell is CaV 1.1 or CaV 1.2, the at least one postsynaptic small molecule used in the composition of the invention is verapamil.

### Postsynpatic peptides (cardiac)

In this embodiment, when the at least one synaptic inhibitor of neuronal transmission is a postsynaptic peptide, it is selected according to the target postsynaptic receptor expressed by a cardiac muscle cell as listed in Table 6.

**Table 6: postsynaptic peptides and cardiac muscle cell as postsynaptic cell**

| **Target postsynaptic receptor** | **Name of postsynaptic peptide** | **Sequence ID** |
|---|---|---|
| mAChR M2 | Muscarinic toxin α | SEQ ID NO:59 or 60 |
| CaV1.1 or CaV1.2 | Calciseptine | SEQ ID NO:25 or 26 |
| | Toxin FS2 | SEQ ID NO:25 or 27 |
| | Vasotab | SEQ ID NO:28 or 29 |
| | Glacontryphan M | SEQ ID NO: 30 or 31 |
| | ω-conotoxin-TxVII | SEQ ID NO: 32 |
| | ω-ctenitoxin-Csla | SEQ ID NO:33 or 34 |
| | U7-ctenitoxin-Pn1b | SEQ ID NO:35 |
| | U9-ctenitoxin-Pnla | SEQ ID NO:36 or 37 |
| | U6-ctenitoxin-Pnla | SEQ ID NO: 36 or 38 |
| | κ-ctenitoxin-Pnla | SEQ ID NO:36 or 39 |
| | ω-ctenitoxin-Pr2a | SEQ ID NO: 40 or 41 |
| | Toxin S4C8 | SEQ ID NO:42 or 43 |
| | Toxin C10S2C2 | SEQ ID NO: 42 or 44 |
| CaV1.2 | ω-theraphotoxin-Ccla | SEQ ID NO:61 |
| RYR2 | Imperacalcin | SEQ ID NO:15 or 16 |
| | Phospholipase A2 imperatoxin-1 (large subunit) | SEQ ID NO:17 or 18 |
| | Heteromtoxin (large subunit) | SEQ ID NO: 17 or 19 |
| | Phospholipase A2 (large subunit) | SEQ ID NO:17 or 20 |
| | Phospholipase A2 | SEQ ID NO:17 or 21 |
| | Insecticidal Toxin LalT1 | SEQ ID NO:22 or 23 |
| | ϕ-Liotoxin-Lw1a | SEQ ID NO:22 or 24 |
| NaV1.5 | β-mammal toxin Css2 | SEQ ID NO:62 or 63 |
| | α-like toxin BmK-M1 | SEQ ID NO:64 or 65 |
| | µ-thomitoxin-Hme1a | SEQ ID NO:55 or 56 |
| | µ-thomitoxin-Hme1b | SEQ ID NO:55 or 57 |
| | µ-thomitoxin-Hme1c | SEQ ID NO: 55 or 58 |
| | b/κ-theraphotoxin-Cg1a | SEQ ID NO:66 or 67 |
| | κ-theraphotoxin-Cg1a | SEQ ID NO:68 or 69 |
| | δ-theraphotoxin-Cgla 1 | SEQ ID NO:70 or 71 |
| | δ-theraphotoxin-Cgla 2 | SEQ ID NO:70 or 72 |
| | δ-theraphotoxin-Cgla 3 | SEQ ID NO:70 or 73 |

Postsynaptic peptides able to bind mAChRs M2 and suitable for the composition of the invention are muscarinic toxin α having the sequence SEQ ID NO: 59, SEQ ID NO:60, or a derivative thereof.

Postsynaptic peptides able to bind CaV 1.1 or CaV 1.2 and suitable for the composition of the invention can be selected from the group comprising or consisting of calciseptine (SEQ ID NO:25 or SEQ ID NO:26), toxin FS2 (SEQ ID NO:25 or SEQ ID NO:27), vasotab (SEQ ID NO:28 or SEQ ID NO:29), glacontryphan M (SEQ ID NO:30 or SEq ID NO:31), ω-conotoxin-TxVII (SEQ ID NO:32), ω-ctenitoxin-Cs1a (SEQ ID NO:33 or SEQ ID NO:34), U7-ctenitoxin-Pn1b (SEQ ID NO:35), U9-ctenitoxin-Pnla (SEQ ID NO:36 or SEQ ID NO:37), U6-ctenitoxin-Pnla (SEQ ID NO:36 or SEQ ID NO:38), κ-ctenitoxin-Pnla (SEQ ID NO:36 or SEQ ID NO:39), ω-ctenitoxin-Pr2a (SEQ ID NO:40 or SEQ ID NO:41), toxin S4C8 (SEQ ID NO:42 or SEQ ID NO:43) and toxin C10S2C2 (SEQ ID NO:42 or SEQ ID NO:44), ω-theraphotoxin-Ccla (SEQ ID NO:61), or derivatives thereof.

In a preferred embodiment, when calciseptine or Toxin FS2 is used in the composition of the invention, it has the sequence SEQ ID NO:25, SEQ ID NO:26, or a derivative thereof.

In a preferred embodiment, when vasotab is used in the composition of the invention, it has the sequence SEQ ID NO:28, SEQ ID NO:29, or a derivative thereof.

In a preferred embodiment, when glacontryphan M is used in the composition of the invention, it has the sequence SEQ ID NO:30, SEQ ID NO:31, or a derivative thereof.

In a preferred embodiment, when ω-conotoxin-TxVII is used in the composition of the invention, it has the sequence SEQ ID NO:32, or a derivative thereof.

In a preferred embodiment, when ω-ctenitoxin-Cs1a is used in the composition of the invention, it has the sequence SEQ ID NO:33, SEQ ID NO:34, or a derivative thereof.

In a preferred embodiment, when U9-ctenitoxin-Pnla, U6-ctenitoxin-Pnla, or κ-ctenitoxin-Pnla is used in the composition of the invention, it has the sequence SEQ ID NO:37, SEQ ID NO:38, SEQ ID NO:39, or a derivative thereof

In a preferred embodiment, when ω-ctenitoxin-Pr2a is used in the composition of the invention, it has the sequence SEQ ID NO:40, SEQ ID NO:41, or a derivative thereof In a preferred embodiment, when toxin S4C8 or toxin C10S2C2 is used in the composition of the invention, it has the sequence SEQ ID NO:43, SEQ ID NO:44, or a derivative thereof.

In an even more preferred embodiment, when the target postsynaptic receptor expressed by a cardiac muscle cell is CaV1.1 or CaV1.2, the at least one postsynaptic peptide used in the composition of the invention is U7-ctenitoxin-Pn1b having the sequence SEQ ID NO:35, or calciseptine having the sequence SEQ ID NO:25 or SEQ ID NO:26, or derivatives thereof.

When more specificity towards the target postsynaptic receptor CaV1.2 is required, the at least one postsynaptic peptide used in the composition of the invention is ω - theraphotoxin-Ccla having the sequence SEQ ID NO:61, or a derivative thereof.

Postsynaptic peptides able to bind RyR2 and suitable for the composition of the invention can be selected from the group comprising or consisting of imperacalcin (SEQ ID NO:15 or SEQ ID NO:16), phospholipase A2 imperatoxin-1 (large subunit) (SEQ ID NO:17 or SEQ ID NO:18), heteromtoxin (large subunit) (SEQ ID NO:17 or SEQ ID NO:19), phospholipase A2 (large subunit) (SEQ ID NO:17 or SEQ ID NO:20), phospholipase A2 (SEQ ID NO:17 or SEQ ID NO:21), insecticidal toxin LaIT1 (SEQ ID NO:22 or SEQ ID NO:23) and Phi-liotoxin LW1a (SEQ ID NO:22 or SEQ ID NO:24), or derivatives thereof.

In a preferred embodiment, when imperacalcin is used in the composition of the invention, it has the sequence SEQ ID NO:16, or a derivative thereof.

In a preferred embodiment, when phospholipase A2 imperatoxin-1 (large subunit), or heteromtoxin (large subunit), or phospholipase A2 (large subunit), or phospholipase A2 is used in the composition of the invention, it has the sequence SEQ ID NO:18, SEQ ID NO:19, SEQ ID NO:20, SEQ ID NO:21, or a derivative thereof.

In a preferred embodiment, when Insecticidal Toxin LalT1 or ϕ-liotoxin LW1a is used in the composition of the invention, it has the sequence SEQ ID NO:23, SEQ ID NO:24, or a derivative thereof.

In an even more preferred embodiment, when the target postsynaptic receptor expressed by a cardiac muscle cell is RyR2, the at least one postsynaptic peptide used in the composition of the invention is insecticidal Toxin LalT1 having the sequence SEQ ID NO:22, SEQ ID NO:23, or a derivative thereof.

Postsynaptic peptides able to bind NaV1.5 and suitable for the composition of the invention can be selected from the group comprising or consisting of β-mammal toxin Css2 (SEQ ID NO:62 or SEQ ID NO:63), α-like toxin BmK-M1 (SEQ ID NO:64 or SEQ ID NO:65), µ-thomitoxin-Hme1a (SEQ ID NO:55 or SEQ ID NO:56), µ-thomitoxin-Hme1b (SEQ ID NO:55 or SEQ ID NO:57), µ-thomitoxin-Hme1c (SEQ ID NO:55 or SEQ ID NO:58), β/κ-theraphotoxin-Cg1a (SEQ ID NO:66 or SEQ ID NO:67), κ-theraphotoxin-Cg1a (SEQ ID NO:68 or SEQ ID NO:69), δ-theraphotoxin-Cgla 1 (SEQ ID NO:70 or SEQ ID NO:71), δ-theraphotoxin-Cgla 2 (SEQ ID NO:70 or SEQ ID NO:72) and δ-theraphotoxin-Cgla 3 (SEQ ID NO:70 or SEQ ID NO:73), or derivatives thereof.

In a preferred embodiment, when β-mammal toxin Css2 is used in the composition of the invention, it has the sequence SEQ ID NO:63, or a derivative thereof.

In a preferred embodiment, when α-like toxin BmK-M1 is used in the composition of the invention, it has the sequence SEQ ID NO:65, or a derivative thereof.

In a preferred embodiment, when µ-thomitoxin-Hme1a or µ-thomitoxin-Hme1b or µ-thomitoxin-Hme1c is used in the composition of the invention, it has the sequence SEQ ID NO:56, SEQ ID NO:57, SEQ ID NO:58, or a derivative thereof.

In a preferred embodiment, when β/κ-theraphotoxin-Cg1a is used in the composition of the invention, it has the sequence SEQ ID NO:67, or a derivative thereof.

In a preferred embodiment, when κ-theraphotoxin-Cg1a is used in the composition of the invention, it has the sequence SEQ ID NO:69, or a derivative thereof.

In a preferred embodiment, when δ-theraphotoxin-Cg1a 1, or δ-theraphotoxin-Cgla 2, or δ-theraphotoxin-Cg1a 3 is used in the composition of the invention, it has the sequence SEQ ID NO:71, SEQ ID NO:72, SEQ ID NO:73, or a derivative thereof.

In a more preferred embodiment, when the target postsynaptic receptor expressed by a cardiac muscle cell is Nav1.5, the at least one postsynaptic peptide used in the composition of the invention is κ-theraphotoxin-Cg1a having the sequence SEQ ID NO:68 or SEQ ID NO:69, or β-mammal toxin Css2 having the sequence SEQ ID NO:62 or SEQ ID NO:63, or derivatives thereof.

### Secretory Gland cell

In another embodiment, when the postsynaptic cell is a secretory gland cell, the at least one postsynaptic inhibitor of cholinergic neuronal transmission used in the composition according to the invention binds to at least one receptor expressed by a secretory gland cell, said receptor being selected from the group comprising or consisting of muscarinic acetylcholine receptors (mAChRs), and more preferably M1 or M3 subtypes mAChRs, α₁ adrenergic receptor and β₁adrenergic receptor.

When two or more postsynaptic inhibitors of cholinergic neuronal transmission (PoNT) are used as ingredients in the composition according to the invention, the receptors expressed by the secretory gland cell and targeted by these PoNT may belong to the same receptor family or to several distinct families of receptors.

### Small molecule (secretory gland)

In this embodiment, when the at least one synaptic inhibitor of neuronal transmission is a postsynaptic small molecule, it is selected according to the target postsynaptic receptor expressed by a secretory gland cell as listed in Table 7.

**Table 7: postsynaptic small molecules and secretory gland cell as postsynaptic cell**

| **Target postsynaptic receptor** | **Name of postsynaptic small molecule** | **MW (g/mol)** |
|---|---|---|
| mAChRs M1 or M3 | Atropine | 289.4 |
| | Scopolamine | 303.3 |
| | Hyoscyamine | 289.4 |
| | Ipratropium | 426.5 |
| | Tropicamide | 284.4 |
| | Flavoxate | 391.5 |
| | Oxybutynin | 394 |
| | Tiotropium | 472 |
| | Cyclopentolate | 291.4 |
| | Tolterodine | 325.5 |
| | Procyclidine | 287.4 |
| | Aclidinium | 564.6 |
| mAChRs M1 | Pirenzepine | 351.4 |
| | Telenzepine | 370.5 |
| | Benzhexol | 351.4 |
| mAChRs M3 | Solifenacin | 480.5 |
| | Darifenacin | 507 |
| | 4-DAM P | 451.4 |
| | DAU-5884 | 351.8 |
| | J-104129 | 500.6 |
| | Zamifenacin | 531.6 |
| α1-adrenergic R | Alfuzosin | 389.5 |
| | Doxazosin | 451.5 |
| | Indoramin | 347.5 |
| | Moxisylyte | 279.4 |
| | Prazosin | 383.4 |
| | Silodosin | 495.5 |
| | Tamsulosin | 408.5 |
| | Terazosin | 387.4 |
| β1-adrenergic R | Acebutolol | 336.4 |
| | Atenolol | 266.3 |
| | Betaxolol | 307.4 |
| | Bisoprolol | 325.4 |
| | Esmolol | 295.4 |
| | Metoprolol | 267.4 |
| | Nebivolol | 405.4 |
| | Vortioxetine | 298.5 |

Several muscarinic receptor subtypes are involved in salivary secretion, serous cells expressing a single population of M3 receptors, and mucous acinar cells expressing both M1 and M3 receptors.

In this embodiment, when the at least one synaptic inhibitor of neuronal transmission is a postsynaptic small molecule that binds mAChRs M1 or M3, it is selected from the group comprising or consisting of atropine, scopolamine, hyoscyamine, ipratropium, tropicamide, flavoxate, oxybutynin, tiotropium, cyclopentolate, tolterodine, procyclidine, aclidinium, or a combination thereof.

In a more preferred embodiment, when the target postsynaptic receptor expressed by a secretory gland cell is mAChRs M1 or M3, the at least one postsynaptic small molecule used in the composition of the invention is oxybutynin.

More specificity toward M1 or M3 mAchR may be required.

In this embodiment, when the at least one synaptic inhibitor of neuronal transmission is a postsynaptic small molecule that binds mAChRs M1, it is selected from the group comprising or consisting of pirenzepine, telenzepine, benzhexol, or a combination thereof.

In a more preferred embodiment, when the target postsynaptic receptor expressed by a secretory gland cell is mAChRs M1, the at least one postsynaptic small molecule used in the composition of the invention is pirenzepine, benzhexol, or a combination thereof.

In this embodiment, when the at least one synaptic inhibitor of neuronal transmission is a postsynaptic small molecule that binds mAChRs M3, it is selected from the group comprising or consisting of solifenacin, darifenacin, 4-DAMP, DAU-5884, J-104129, zamifenacin, or a combination thereof.

In a more preferred embodiment, when the target postsynaptic receptor expressed by a secretory gland cell is mAChRs M3, the at least one postsynaptic small molecule used in the composition of the invention is darifenacin.

Adrenergic receptors (ARs) are a class of G protein-coupled receptors and associate with adenylyl cyclase. ARs include four general types (α1, α2, β1 and β2), which are found in different effector tissues. In salivary gland, α1-adrenergic receptors induce an increase in the cytosolic concentration of calcium ([Ca2⁺]i) and stimulate salivary fluid secretion. α1-adrenergic pathway is also active in lacrimal glands.

In this embodiment, when the at least one synaptic inhibitor of neuronal transmission is a postsynaptic small molecule that binds α1-adrenergic receptor, it is selected from the group comprising or consisting of alfuzosin, doxazosin, indoramin, moxisylyte, prazosin, silodosin, tamsulosin, terazosin, or a combination thereof.

In a more preferred embodiment, when the target postsynaptic receptor expressed by a secretory gland cell is α1-adrenergic receptor, the at least one postsynaptic small molecule used in the composition of the invention is silodosin.

In salivary glands, β1-adrenergic receptors are responsible of viscous secretions.

In this embodiment, when the at least one synaptic inhibitor of neuronal transmission is a postsynaptic small molecule that binds β1-adrenergic receptor, it is selected from the group comprising or consisting of acebutolol, atenolol, betaxolol , bisoprolol, esmolol, metoprolol, nebivolol, vortioxetine, or a combination thereof.

In a more preferred embodiment, when the target postsynaptic receptor expressed by a secretory gland cell is β1-adrenergic receptor, the at least one postsynaptic small molecule used in the composition of the invention is bisoprolol.

### Postsynaptic Peptide (secretory gland)

In this embodiment, when the at least one synaptic inhibitor of neuronal transmission is a postsynaptic peptide, it is selected according to the target postsynaptic receptor expressed by a secretory gland cell as listed in Table 8.

**Table 8: postsynaptic peptides and secretory gland cell as postsynaptic cell**

| **Target postsynaptic receptor** | **Name of postsynaptic peptide** | **Sequence ID** |
|---|---|---|
| mAChrs M1 | Muscarinic toxin 1 | SEQ ID NO:59 or 74 |
| | Muscarinic toxin 2 | SEQ ID NO:59 or 75 |
| | Muscarinic toxin 3 | SEQ ID NO:59 or 76 |
| | Muscarinic toxin 7 | SEQ ID NO:59 or 77 |
| | Muscarinic toxin α | SEQ ID NO:59 or 60 |
| mAChrs M3 | Muscarinic toxin α | SEQ ID NO:59 or 60 |
| α1-adrenergic R | Muscarinic toxin 1 | SEQ ID NO:59 or 74 |
| | Muscarinic toxin 3 | SEQ ID NO:59 or 76 |
| | Muscarinic toxin α | SEQ ID NO:59 or 60 |
| | Adrenergic toxin ρ-elapitoxin-Dp1a | SEQ ID NO:59 or 78 |
| | Toxin AdTx1 | SEQ ID NO:59 or 79 |
| β1-adrenergic R | Beta-cardiotoxin | SEQ ID NO:80 or 81 |

Postsynaptic peptides able to bind mAChrs M1 suitable for the composition of the invention can be selected from the group comprising or consisting of muscarinic toxin 1 (SEQ ID NO:59 or SEQ ID NO:74), muscarinic toxin 2 (SEQ ID NO:59 or SEQ ID NO:75), muscarinic toxin 3 (SEQ ID NO:59 or SEQ ID NO:76), muscarinic toxin 7 (SEQ ID NO:59 or SEQ ID NO:77) and muscarinic toxin α (SEQ ID NO:59 or SEQ ID NO:60), or derivatives thereof.

Consensus sequence of muscarinic toxin α, muscarinic toxin 1, muscarinic toxin 2, muscarinic toxin 3 and muscarinic toxin 7 (SEQ ID NO:59) has been defined relying on the sequences of the proteins having the following accession number in Uniprot database: P80494, Q9PSN1, P81030, P86419, P80495, P85092, P25518, P17696, P82463, P60234, P18328, Q8QGR0, P81031.

In a more preferred embodiment, when the postsynaptic receptor on the surface of a secretory gland cell is mAChrs M1, the at least one postsynaptic peptide used in the composition of the invention is muscarinic toxin 7 having the sequence SEQ ID NO:59, SEQ ID NO:77, or a derivative thereof.

In this embodiment, when the at least one synaptic inhibitor of neuronal transmission is a postsynaptic peptide that binds mAChrs M3, the at least one postsynaptic peptide used in the composition of the invention is muscarinic toxin α having the sequence SEQ ID NO:59, SEQ ID NO:60, or a derivative thereof.

Postsynaptic peptides able to bind α1-adrenergic receptor suitable for the composition of the invention can be selected from the group comprising or consisting of muscarinic toxin 1 (SEQ ID NO:59 or SEQ ID NO:74), muscarinic toxin 3 (SEQ ID NO:59 or SEQ ID NO:76), muscarinic toxin α (SEQ ID NO:59 or SEQ ID NO:60), adrenergic toxin ρ-elapitoxin-Dpla (SEQ ID NO:78), toxin AdTx1 (SEQ ID NO:79), or derivatives thereof.

Consensus sequence of muscarinic toxin α, muscarinic toxin 1, muscarinic toxin 3, adrenergic toxin ρ-elapitoxin-Dp1a and toxin AdTx1 (SEQ ID NO:59) has been defined relying on the sequences of the proteins having the following accession number in Uniprot database: P80494, Q9PSN1, P81030, P86419, P80495, P85092, P25518, P17696, P82463, P60234, P18328, Q8QGR0, P81031.

In a more preferred embodiment, when the postsynaptic receptor on the surface of a secretory gland cell is α1-adrenergic receptor, the at least one postsynaptic peptide used in the composition of the invention is adrenergic toxin ρ-elapitoxin-Dp1a having the sequence SEQ ID NO:59 or SEQ ID NO:78, or toxin AdTx1 having the sequence of SEQ ID NO:59 or SEQ ID NO:79, or derivatives thereof.

Postsynaptic peptides able to bind β1-adrenergic receptor suitable for the composition of the invention can be selected from the group comprising or consisting of beta-cardiotoxin (SEQ ID NO:80 or SEQ ID NO:81).

Consensus sequence of β-cardiotoxin (SEQ ID NO:80) has been defined relying on the sequences of the proteins having the following accession number in Uniprot database: Q69CK0, Q53B46, Q2VBN8, Q2VBN5, Q2VBN7, Q2VBN4.

In this embodiment, when the at least one synaptic inhibitor of neuronal transmission is a postsynaptic peptide that binds β1-adrenergic receptor, the at least one postsynaptic peptide used in the composition of the invention is β-cardiotoxin having the sequence SEQ ID NO:80, SEQ ID NO:81, or a derivative thereof.

### Multi-specific post-synaptic inhibitors

In other embodiments, postsynaptic inhibitors of neuronal transmission can be chosen among small molecules and peptides that show the ability to inhibit multiple molecular targets. Such "multi-specific postsynaptic inhibitors" have an activity on at least one of the molecular target described above (namely, nAChRs ((α1)₂β1δε or (α1)₂β1δγ), mAChRs M1 or mAChRs M2 or mAChRs M3, α1-adrenergic receptor, β1-adrenergic receptor, RYR1 or RYR2, CaV1.1 or CaV1.2, NaV1.4 or NaV1.5). Of note, the multispecificity can be advantageous to the invention: for example, the use of small molecules inhibiting largely NaVs could reduce the pain associated to the procedure or condition (since NaV1.7 is notably involved in nociception).

In some embodiments, multi-specific postsynaptic inhibitors can be selected from the small molecules listed in Table 9.

**Table 9: multi-specific postsynaptic small molecules**

| **Name of postsynaptic small molecule** | **MW (g/mol)** |
|---|---|
| Niguldipine | 609.7 |
| Oxodipine | 359.4 |
| Fendiline | 315.4 |
| Mibefradil | 459.6 |
| Bepridil | 366.5 |
| Flunarizine | 404.5 |
| Fluspirilene | 475.6 |
| Saxitoxin | 299.3 |
| Neosaxitoxin | 315.3 |
| Gonyautoxin | 395.4 |
| Decarbamoylsaxitoxin | 256.3 |
| Tetrodotoxin | 319.3 |
| Quinidine | 324.4 |
| Ajmaline | 326.4 |
| Procainamide | 235.3 |
| Disopyramide | 339.5 |
| Mexiletine | 179.3 |
| Phenytoin | 252.3 |
| Tocainide | 192.3 |
| Encaidine | 352.5 |
| Flecaidine | 414.3 |
| Propaferone | 341.4 |
| Moricizine | 427.5 |
| Lorcainide | 370.9 |
| Lidocaine | 234.3 |
| Benzocaine | 165.2 |
| Dimethocaine | 278.4 |
| Prilocaine | 220.3 |
| Procaine | 236.3 |
| Tetracaine | 264.4 |
| Carbamazepine | 236.3 |
| Oxcarbazepine | 252.3 |
| Eslicarbazepine | 296.3 |
| Lamotrigine | 256.1 |
| Cannabidiol | 314.5 |
| Diphenhydramine | 255.4 |
| Dicycloverine | 309.5 |
| Benzatropine | 307.4 |
| Biperiden | 348 |
| Ranolazine | 427.5 |
| Chlorpromazine | 318.9 |
| Dimethindene | 292.4 |
| Gallamine | 423.6 |
| Arotinolol | 371.5 |
| Carvedilol | 406.5 |
| Labetalol | 328.4 |
| Phenoxybenzamine | 303.8 |
| Phentolamine | 281.4 |
| Tiamenidine | 215.7 |
| Tolazoline | 160.2 |
| Trimazosin | 435.5 |
| Gabapentin | 171.2 |
| Pregabalin | 159.2 |

In a more preferred embodiment, the at least one postsynaptic multi-specific small molecule used in the composition of the invention is lidocaine.

In other embodiments, multi-specific postsynaptic inhibitors can be selected from the peptides listed in Table 10, or derivatives thereof.

**Table 10: multi-specific postsynaptic peptides**

| **Name of postsynaptic peptide** | **Sequence ID** |
|---|---|
| ω-agatoxin-IIIA | SEQ ID NO:82 |
| Calcicludine | SEQ ID NO:83 |
| ω-filistatoxin-Kh1a | SEQ ID NO:84 |
| β-theraphotoxin-Gr1a | SEQ ID NO:85 |
| β -theraphotoxin-Gr1b | SEQ ID NO:86 |
| κ-theraphotoxin-Gr2c | SEQ ID NO:87 |
| M-theraphotoxin-Gr1a | SEQ ID NO:88 |
| Taicatoxin, alpha-neurotoxin-like component | SEQ ID NO:89 |
| β/ω-theraphotoxin-Tp1a | SEQ ID NO:90 |
| β/ω-theraphotoxin-Tp2a | SEQ ID NO:91 |
| β/κ-theraphotoxin-Cg2a | SEQ ID NO:92 |
| µ-theraphotoxin-Hhn1b (1-2-3) | SEQ ID NO:93 |
| µ-theraphotoxin-Hhn1a | SEQ ID NO:94 |

In a more preferred embodiment, the at least one postsynaptic multi-specific peptide used in the composition of the invention is β/ω-theraphotoxin-Tp2a having the sequence SEQ ID NO:91, or a derivative thereof.

The invention further provides a cosmetic composition comprising the composition of the invention comprising at least one postsynaptic inhibitor of cholinergic neuronal transmission and a botulinum neurotoxin. Said cosmetic composition may further comprise a cosmetically acceptable excipient or diluent.

The invention further provides a pharmaceutical composition comprising the composition of the invention comprising at least one postsynaptic inhibitor of cholinergic neuronal transmission and a botulinum neurotoxin. Said pharmaceutical composition may further comprise a pharmaceutically acceptable excipient, diluent, carrier, salt and/or additive.

### Embodiment & other aspect of invention

BoNT has been reported to have direct effects including inhibition or blockage of the cholinergic neuromuscular or the cholinergic autonomic innervation of secretory glands and smooth muscles.

The composition of the invention enables performing experiments to modulate the pharmacodynamic profile of BoNT.

### PoNTs with fast onset

Getting fast onset may be particularly interesting for fast relief of pain related to muscle contraction.

Surprisingly, the inventors have observed that the "early effects" of myorelaxation of the composition of the invention are stronger than the "early effect" of individual compounds (namely the BoNT and the PoNT) administered independently. There is thus a synergistic effect of the composition. By "early effects" is meant a myorelaxation effect that is produced before the effect of BoNT, that is within the first 24h after BoNT administration.

In preferred embodiments, the Postsynaptic inhibitor of Neural Transmission (PoNT) used in the composition of the invention is a fast onset postsynaptic peptide and/or fast onset postsynaptic small molecule. By "fast-onset" is meant a PoNT that produces effects more rapidly than those produced by, for example, a botulinum neurotoxin, such as for example BoNT/A or BoNT/B or BoNT/E. For example, the effects of the fast-onset PoNT can be visible within 12 hours, preferably within 6 hours, more preferably within 2 hours, and most preferably within 1 hour.

In other preferred embodiments, the Postsynaptic inhibitor of Neural Transmission (PoNT) used in the composition of the invention is a very fast onset postsynaptic peptide or small molecule. By "very fast onset" is meant a PoNT that produces effects within less than 45 minutes, preferably less than 30 minutes, more preferably less than 20 minutes and most preferably less than 10 minutes.

### PoNTs that increase BoNT duration of action

More surprisingly, the inventors discovered that the addition of at least one postsynaptic inhibitor of cholinergic neuronal transmission, i.e. postsynaptic peptide or postsynaptic small molecule, to botulinum neurotoxin compositions extends the duration of action of botulinum neurotoxin. As for example, the usual duration, after an intramuscular injection, of the action of BoNT/A is typically about 3 to 4 months in human. By "extending the duration of action" of botulinum neurotoxin, is meant the action of the botulinum toxin lasts longer than by, for example, the action of a botulinum neurotoxin of type A. The cessation of action of the composition of the invention occurs, for example, 1 week, 2 week, 3 weeks, 1 month, 2 months, or more, later as compared to the cessation of action of a composition comprising only BoNT.

This specific advantage of the invention may be particularly interesting for reducing the number of injections required to achieve a desired effect. Another advantage is that by increasing the time between two injections, the likelihood of generating anti-BoNT antibodies by the immune system of the individual receiving the composition of the invention is also reduced.

### PoNTs that increase the intensity

Surprisingly, the inventors discovered that the administration of at least one postsynaptic inhibitor of cholinergic neuronal transmission, i.e. postsynaptic peptide or postsynaptic small molecule, together with botulinum neurotoxin compositions enhances the intensity of action of botulinum neurotoxin. By "intensity of action" is meant the highest myorelaxing effects observable for a specific dose of BoNT.

In preferred embodiment, the Postsynaptic inhibitor of Neural Transmission (PoNT) used in the composition of the invention is exhibiting a synergistic effect that is enhanced by at least 10%, preferably at least 25%, more preferably at least 50% than the effects of individual compounds of the composition administered independently.

By enhancing the intensity of action of BoNT, the composition of the invention may improve the aesthetic result and the relief of disabling and painful conditions.

### PoNTs with fast onset, that decrease BoNT onset, that increase BoNT intensity of action, and that increase duration of action

For some PoNTs, i.e. postsynaptic peptide or postsynaptic small molecule, having fast or very fast onset it is possible to modulate the effect of BoNT composition by accelerating the onset of action and extending the duration of action and enhancing the intensity of action of a botulinum neurotoxin composition.

### Triple combo for fast and persistent onset

Depending on the intended use, in case the PoNT has a duration of action ending before the botulinum neurotoxin maximum effect is obtained, then the composition of the invention may further comprise a second PoNT having an intermediate onset of action. By "intermediate onset of action" is meant PoNT that produces effects more slowly than a fast-onset PoNT. The advantage is to obtain rapid, sustained and prolonged maximum effects of the composition of the invention, without significant decrease of these effects over the time.

In a preferred embodiment, the composition of the invention comprises at least one fast-onset PoNT, at least one intermediate-acting PoNT and a botulinum neurotoxin.

### Simultaneous administration

In all embodiments, the at least one PoNT and the botulinum neurotoxin are administered in combination. The terms "in combination", "in combination with", "codelivery", and "administered together with" are equivalent and may be used interchangeably. In the context of the administration of two or more components to a subject refers to simultaneous administration of the two or more components, such as at least one postsynaptic inhibitor of cholinergic neuronal transmission and the botulinum neurotoxin composition.

Simultaneous administration of PoNT and BoNT is defined as administering at least one PoNT and BoNT at the same anatomic sites within a short time period such as 5, 10, 15, 20, 25 or 30 minutes, or within a longer time period such as 60, 120, 240, 360 or 720 minutes, using similar or different routes of administration, and wherein PoNT and BoNT are combined or not in the same composition or in the same container.

The use of the term "in combination with" does not restrict the order in which PoNT and BoNT are administered to a subject.

### Simultaneous injections

All the above mentioned effects when using the composition of the invention are obtained when the at least one PoNT, i.e. postsynaptic peptide or postsynaptic small molecule, and the botulinum neurotoxin are injected simultaneously.

### Non-simultaneous injection

In an alternative embodiment, the at least one postsynaptic inhibitor of cholinergic neuronal transmission can be injected first and then a second injection with botulinum neurotoxin is carried out. In this embodiment, the botulinum neurotoxin injection must occur before the end of duration of action of the at least one postsynaptic inhibitor of cholinergic neuronal transmission. In other word, non-simultaneous injection of PoNt and botulinum neurotoxin may occurs with the proviso that the botulinum neurotoxin is administered before the decay of PoNT activity occurs, and optionally the botulinum neurotoxin is administered after the onset of PoNT activity.

In another alternative embodiment, the botulinum neurotoxin can be injected first and then a second injection with the at least one postsynaptic inhibitor of cholinergic neuronal transmission is carried out. In this embodiment, the at least one post-synaptic inhibitor of neuronal transmission must be injected before the botulinum neurotoxin effect reaches its plateau.

### Topical administration

As compared to the 150 KDa of BoNT, the PoNTs used in the composition of the invention have a low molecular weight. In the context of cosmetic treatments, it could be interesting to use different types of applications for each of the components of the compositions of the invention. For example, the at least one PoNT may be delivered using topical application.

In another embodiment, the at least one postsynaptic inhibitor of cholinergic neuronal transmission can be applied topically on skin close to the target tissue of interest and the botulinum neurotoxin is injected at the target tissue of interest. In this embodiment, the botulinum neurotoxin injection must occur before the end of duration of action of the at least one postsynaptic inhibitor of cholinergic neuronal transmission.

### Injection schedule

It is also necessary that the injection schedule is adapted to the pharmacodynamic profile of the PoNT so that it can interact with the botulinum neurotoxin at the synapse level in the target tissue.

A second object of the invention relates to a method for enhancing the effect of a botulinum neurotoxin composition, comprising the addition of at least one postsynaptic inhibitor of cholinergic neuronal transmission, i.e. postsynaptic peptide or postsynaptic small molecule, to the botulinum neurotoxin composition.

By "enhancing the effect of a botulinum neurotoxin composition" is meant accelerating the onset of action and /or extending the duration of action and/or enhancing the intensity of action of the botulinum neurotoxin composition.

Thus, the invention relates to a method for accelerating the onset and/or extending the duration of action and/or enhancing the intensity of action of a botulinum neurotoxin composition, comprising the addition of at least one postsynaptic inhibitor of cholinergic neuronal transmission, i.e. postsynaptic peptide or postsynaptic small molecule, to the botulinum neurotoxin composition.

### Onset

By "accelerating the onset" of action of botulinum neurotoxin composition is meant obtaining the relaxing of a muscle faster than that obtained with BoNT alone.

Preferably, the onset of action of the botulinum neurotoxin composition comprising the at least one postsynaptic inhibitor of cholinergic neuronal transmission, i.e. postsynaptic peptide or postsynaptic small molecule, occurs 50%, preferably 75%, more preferably 90% earlier as compared to the duration of action of a botulinum neurotoxin composition comprising BoNT alone (i.e. not comprising any postsynaptic inhibitor of cholinergic neuronal transmission).

Since the same botulinum neurotoxin is used in both compositions, the botulinum neurotoxin composition comprising BoNT alone serves as relevant control for the comparison.

### Duration

By "extending the duration" of action of botulinum neurotoxin composition is meant that the at least one postsynaptic inhibitor of cholinergic neuronal transmission, i.e. postsynaptic peptide or postsynaptic small molecule, slows down the recovery from BoNTs-induced myorelaxation. In other words, the duration of muscle relaxation following the intramuscular injection of the composition of the invention may be considered as "long-recovery".

Preferably, the duration of action of the botulinum neurotoxin composition comprising the at least one postsynaptic inhibitor of cholinergic neuronal transmission, i.e. postsynaptic peptide or postsynaptic small molecule, is extended by 10%, 25%, 50%, 100% or more as compared to the duration of action of a botulinum neurotoxin composition comprising BoNT alone (i.e. not comprising any postsynaptic inhibitor of cholinergic neuronal transmission).

Since the same botulinum neurotoxin is used in both compositions, the botulinum neurotoxin composition comprising BoNT alone serves as relevant control for the comparison.

### Intensity

In some embodiments, a third effect of the at least one postsynaptic inhibitor of cholinergic neuronal transmission may consist in increasing the maximum intensity of action of the botulinum toxin. Preferably, the maximum intensity of action is enhanced by at least 2 %, preferably at least 5%, more preferably at least 10% as compared to the maximum intensity of action of a composition containing only botulinum neurotoxin (i.e. not comprising any postsynaptic inhibitor of cholinergic neuronal transmission).

Since the same botulinum neurotoxin is used in both compositions, the botulinum neurotoxin composition comprising BoNT alone serves as relevant control for the comparison.

This third effect may be particularly interesting to achieve better relief of patients, for example in severe dystonia. Another advantage is the fact that the composition of the invention can achieve satisfactory effects with lower doses of BoNT, thus reducing the risks of BoNT over-dosage-related toxicity (especially in Parkinson patients requiring multiple injections in various parts of the body).

### Onset, intensity and duration

In some embodiments, the at least one postsynaptic inhibitor of cholinergic neuronal transmission, i.e. postsynaptic peptide or postsynaptic small molecule, has three effects on the botulinum neurotoxin composition: accelerating the onset and extending the duration of action and enhancing the intensity of action of the botulinum neurotoxin composition.

The composition of the invention can further comprise other components suitable for aesthetic or therapeutic uses.

### Formulation

A third object of the invention is a cosmetic or pharmaceutical composition comprising the composition of the invention and one or more dermatologically or pharmaceutically acceptable carriers, such as, but not limited to an albumin, preferably a human serum albumin or a recombinant human albumin, a non-reducing di- or tri-saccharide, a nonionic surfactant, and any other salt and/or additive suitable for the intended uses.

Compositions of this invention are preferably in a form that permits subcutaneous, intramuscular, implant and/or topical administration. Intramuscular and subcutaneous administrations are preferred.

Compositions of the invention suitable for injection in subjects or patients, (i.e., humans or other mammals in need of the particular treatment) may be in the form of solutions, suspensions, emulsions, or dried powder which is dissolved or suspended in a suitable vehicle prior to use. The term "in need" is meant to include both pharmaceutical- or health- related needs (e.g., treating conditions related to dystonia or spasticity), as well as cosmetic and subjective needs (e.g., altering or improving the appearance such as treatment of facial wrinkles).

When postsynaptic peptide is to be delivered locally, it may be formulated as a cream, a foam, a gel, a lotion or an ointment (e.g. for topical application), or for sub-dermal injection. Topical delivery means may include transdermal delivery (e.g. via an adhesive patch, iontophoresis or ultrasound device).

The compositions of the invention are suitable for local delivery. By "local delivery" is meant that the site of action is at, or near to the site of application of the composition. As a non-limiting example, a composition comprising at least one PoNT and a botulinum neurotoxin injected into a muscle to be relaxed would be local delivery to that muscle by intramuscular injection.

### Mixing of PoNT and BoNT

In preferred embodiments, the compositions are prepared by mixing the botulinum toxin (either containing the associated non-toxin proteins or without associated non-toxin proteins) with the at least one postsynaptic inhibitor of cholinergic neuronal transmission, and usually with one or more additional pharmaceutically acceptable carriers or excipients. In their simplest form, they may contain an aqueous pharmaceutically acceptable diluent, such as buffered saline. However, the compositions may contain other ingredients typically found in injectable pharmaceutical or cosmeceutical compositions, including a dermatologically or pharmaceutically acceptable carrier, vehicle or medium that is compatible with the tissues to which it will be applied. The term "dermatologically or pharmaceutically acceptable," as used herein, means that the compositions or components thereof so described are suitable for use in contact with these tissues or for use in patients in general without undue toxicity, incompatibility, instability, allergic response, and the like. When appropriate, compositions of the invention may comprise any ingredient conventionally used in the fields under consideration, and particularly in cosmetics and dermatology.

### Administration into skeletal muscles

The pharmaceutical compositions of the invention are suitable for injection, into or in close vicinity of one or more of the following muscles, for example, the corrugator supercilii, procerus, occipitofrontalis, nasalis, orbicularis oris, depressor anguli oris, platysma, sternohyoid, serratus anterior, rectus abdominis, external oblique, tensor fasciae latae, brachioradialis, Iliacus, psoas major, pectineus, adductor longus, sartorius, gracillis, vastus lateralis, rectus femoris, vastus medialis, tendon of quadriceps femoris, patella, gastrocnemius, soleus, tibia, fibularis longus, tibialis anterior, patellar ligament, iliotibial tract, hypothenar muscles, thenar muscles, flexor carpi ulnaris, flexor digitorum superficialis, palmaris longus, flexor carpi radials, brachioradialis, pronator teres, brachialis, biceps brachii, triceps brachii, pectoralis major, deltoid, trapezius, sternocleidomastoid, masseter, orbicularis oculi, temporalis, epicranial aponeurosis, teres major, extensor digitorum, extensor carpi ulnaris, anconeus, abductor policis longus, plantaris, calcanel tendon, soleus, adductor magnus, gluteus maximas, gluteus medius, latissimus dorsi, infraspinatus, and combinations thereof, and the like.

### Administration into smooth muscles

Smooth muscles suitable for administration of compositions of the invention can comprise any of walls of blood vessels, walls of stomach, ureters, intestines, in the aorta (tunica media layer), iris of the eye, prostate, gastrointestinal tract, respiratory tract, small arteries, arterioles, reproductive tracts (both genders), veins, glomeruli of the kidneys (called mesangial cells), bladder (detrusor), uterus, arrector pili of the skin, ciliary muscle, sphincter (esophagus, anus), trachea, bile ducts, and the like.

### Cardiac

The pharmaceutical compositions of the invention are suitable for injection into the epicardial fat pads such as the sinus node (right superior and inferior pulmonary vein (PV)) fat pad and the atrioventricular (AV) node (inferior vena cava-left atrium) fat pad.

### Secretory Glands

The pharmaceutical composition of the invention can be injected directly into the salivary glands, to reduce saliva production, or into the submandibular gland (below the floor of the mouth) and the parotid gland (behind the jaw), or into the vicinity of eccrine sweat glands of the hand and foot palms, and axillary.

### Repeated injections

More than one injection and/or sites of injection may be necessary to achieve the desired result. The frequency and the amount of the at least one postsynaptic inhibitor of cholinergic neuronal transmission and the botulinum toxin used in the composition of the invention can be determined by the skilled artisan, based on the nature and location of the particular area being treated, using for example photographs, scanning, MRI, electromyograms, or the like.

### Injection procedure

Injection of the composition of the invention can be carried out by syringe, catheters, needles and other means for injecting. The injection can be performed on any area of a body that needs to be treated, including but not limited to, face, neck, torso, arms, hands, legs and feet. The injection can be into any position in the specific area such as epidermis, dermis, subcutaneous layer, fat or muscle.

### Route

The route and dosage for administration of the composition of the invention can be selected based upon criteria such as the solubility characteristics of the postsynaptic inhibitor of cholinergic neuronal transmission and/or the botulinum neurotoxin as well as the intensity and scope of the cosmetic or therapeutic condition being treated.

### Dosage

In injectable compositions of the invention, useful dosages for the at least one postsynaptic inhibitor of cholinergic neuronal transmission used is about 0.01 ng/kg to 500 µg/kg, preferably about 1 to 80 µg/kg, and more preferably about 4 to 50 µg/kg of body weight of the patient to be treated.

The choice of dosages for the at least one postsynaptic inhibitor of cholinergic neuronal transmission may also relies on the LD50 (the median lethal dose which kills 50 percent of the test population), which is specific for each PoNT. In this case, useful dosages for the at least one postsynaptic inhibitor of cholinergic neuronal transmission used is about 1% to 50% of the LD50, preferably about 5% to 30% of the LD50, more preferably about 10% to 20% of the LD50.

In injectable compositions of the invention, a useful dose range for the botulinum neurotoxin used is about 1 to 1000 Units, preferably about 100 to 500 Units, preferably about 50 to 200 Units, and more preferably about 20 to 100 Units to be treated.

By "Unit" is meant an amount of active BoNT standardized to have equivalent neuromuscular blocking effect as a Unit of commercially available botulinum neurotoxin of type A. It is well known that units of different BoNT/A products do not have equivalent potency and therefore are not interchangeable. While all forms of type A toxins have identical mechanisms of action, the theoretical numbers/amount of active 150 kDa molecules in a vial varies by manufactured product and this variation may have a relative relationship to the LD50 (the median lethal dose which kills 50 percent of the test population). The LD50 may be expressed, for example, in units per mL and is proprietary for each company product, defining the potency units for those products. It may be necessary to calibrate the toxin units against a commercial reference product.

In some embodiments, the botulinum neurotoxin can be administered in a total dose of between 5 to 1000 U.

In some embodiments, the botulinum neurotoxin can be administered in a dose per injection of between 1 U to 500 U, preferably between 10 U to 100 U, more preferably between 20 U to 80 U. Most preferably, the botulinum neurotoxin can be administered in a dose per injection of between 20 U to 50 U.

The dosage of botulinum neurotoxin can also be expressed in protein amount. For example, in certain embodiments, the botulinum neurotoxin can be administered in an amount of between about 6pg to 50ng, preferably between 10 pg to 45 ng, preferably between 20 pg to 30 ng, more preferably between 100 pg to 15 ng/dose.

### Formulations

In terms of their form, compositions of this invention may include solutions, emulsions (including micro- or nano-emulsions), suspensions, gels, powders, or other typical solid or liquid compositions used for administration to muscle and other tissues where the compositions may be used. In preferred embodiments, the compositions of the invention are present in low- viscosity, sterile formulations suitable for injection with a syringe. As used herein, the terms compositions and formulations are essentially interchangeable when referring to the compositions and formulations according to the present invention. The compositions of the invention may be in the form of a lyophilized powder that is reconstituted using a pharmaceutically acceptable liquid diluent prior to injection. The compositions of the invention may contain, in addition to at least one postsynaptic inhibitor of cholinergic neuronal transmission and a botulinum neurotoxin, other ingredients typically used in such products, such as antimicrobials, hydration agents, tissue bulking agents or tissue fillers, preservatives, emulsifiers, natural or synthetic oils, solvents, surfactants, detergents, gelling agents, antioxidants, fillers, thickeners, powders, viscosity-controlling agents and water, and optionally including anesthetics, anti-itch actives, botanical extracts, conditioning agents, minerals, polyphenols, silicones or derivatives thereof, vitamins, and phytomedicinals.

The injectable compositions according to this invention may be in the form of controlledrelease or sustained-release compositions which comprise at least one postsynaptic inhibitor of cholinergic neuronal transmission, i.e. postsynaptic peptide or postsynaptic small molecule, and a botulinum toxin encapsulated or otherwise contained within a material such that they are released within the tissue in a controlled manner over time. The composition comprising the botulinum neurotoxin and postsynaptic inhibitor of cholinergic neuronal transmission may be contained within matrixes, liposomes, vesicles, microcapsules, microspheres and the like, or within a solid particulate material, all of which is selected and/or constructed to provide release of the botulinum toxin over time. The at least one postsynaptic inhibitor of cholinergic neuronal transmission, i.e. postsynaptic peptide or postsynaptic small molecule, and a botulinum toxin may be encapsulated together (i.e., in the same capsule) or separately (i.e., in separate capsules).

### Local administration

Botulinum toxin formulations according to the invention can be delivered by injection (typically using a syringe) to muscles underlying the skin, or to glandular structures within the skin, in an effective amount to produce paralysis, produce relaxation, alleviate contractions, prevent or alleviate spasms, reduce glandular output, alleviate pain due to muscle contraction, or other desired effects. Local delivery of the botulinum toxin in this manner could afford dosage reductions, reduce toxicity and allow more precise dosage optimization for desired effects relative to injectable or implantable materials.

### Effective amount

The compositions of the invention are administered to deliver an effective amount, preferably a therapeutically or aesthetically effective amount, of the botulinum toxin. The term "effective amount" or "therapeutically or aesthetically effective amount" as used herein means an amount of a botulinum toxin as defined above that is sufficient to produce the desired muscular relaxation or other biological or aesthetic effect, but that implicitly is a safe amount, i.e., one that is low enough to avoid serious side effects. Desired effects include the relaxation of certain skeletal muscles with the aim of, for instance, decreasing excessive muscle tone (in the case of neuromuscular disorders such as dystonia) or decreasing the appearance of fine lines and/or wrinkles, especially in the face, or adjusting facial appearance in other ways such as widening the eyes, lifting the corners of the mouth, or smoothing lines that fan out from the upper lip, or the general relief of muscular tension. The last-mentioned effect, general relief of muscular tension, can be effected in the face or elsewhere. Additional desired effects are related to the relaxation of smooth muscle, with the goal of decreasing abnormal contractions; such abnormal contractions arise in patients suffering from overactive bladder, for example. Other desired effects comprise the decrease of neuronal stimulation of cardiac muscle. Pathological neuronal stimulations of cardiac muscle cells are related to heart disease such as atrial fibrillation, justifying the need to diminish these stimulations. Supplemental desired effects concern the reduction of secretory gland stimulation, in pathologies such as hyperhidrosis or sialorrhea.

The compositions of the invention may contain an appropriate effective amount of the botulinum toxin for application as a single-dose treatment, or may be more concentrated, either for dilution at the place of administration or for use in multiple applications. Through the use of the at least one postsynaptic inhibitor of cholinergic neuronal transmission, i.e. postsynaptic peptide or postsynaptic small molecule, a botulinum toxin can be administered by injection to a subject for treating conditions such as wrinkles, undesirable facial muscle or other muscular spasms, hyperhidrosis, acne, or conditions elsewhere in the body in which relief of muscular ache or spasms is desired. The compositions of the invention are particularly suited for the treatment of fine lines, such as facial fine lines, and glabellar lines, also known as "frown lines" in the face of a subject. The botulinum toxin is administered by injection to muscles or to other skin-associated or other effector tissue structures. The administration may be made, for example, to the legs, shoulders, back (including lower back), axilla, palms, feet, neck, face, groin, dorsa of the hands or feet, elbows, upper arms, knees, upper legs, buttocks, torso, pelvis, or any other parts of the body where administration of the botulinum toxin is desired.

### Indications

### Aesthetic

A fourth object of the invention is a cosmetic composition for use in reducing wrinkles, lines, such as glabellar lines, or furrows, muscle volume for aesthetic purposes (such as masseter or calf), hypertrophic scars and other dermatological conditions in an individual in need thereof, said composition comprising at least one postsynaptic inhibitor of cholinergic neuronal transmission, i.e. postsynaptic peptide or postsynaptic small molecule, and a botulinum toxin, and a cosmetically acceptable diluent for injection.

### Therapeutic

Administration of the injectable botulinum toxin-containing composition of the invention may also be carried out to treat other conditions, including any condition for which prevention of synaptic transmission or prevention of acetylcholine release, or prevention of the release of other neurotransmitters, would confer a therapeutic benefit.

For example, the conditions that may be treated by the compositions according to the invention include, without limitation, dystonia and spasticity. The composition of the invention may also be used for treatment of other conditions for which administration of botulinum toxin by injection has been suggested or performed, such as for example pain related to muscle contraction, overactive bladder, rhinitis, sinusitis, acne, dystonia, dystonic contractions (whether subjective or clinical), hyperhidrosis (whether subjective or clinical), and hypersecretion of one or more secretory glands controlled by the cholinergic nervous system.

A fifth object of the invention is a pharmaceutical composition for use in treating movement disorder, dystonia, cervical dystonia, spasmodic torticollis, focal dystonia, focal hand dystonia, blepharospasm, eyelid disorder, strabismus, spasticity, cerebral palsy, focal spasticity, limb spasticity, spasms, hemifacial spasm, tremors, tics, bruxism, apraxia and freezing of gait, said composition comprising at least one postsynaptic inhibitor of cholinergic neuronal transmission, i.e. postsynaptic peptide or postsynaptic small molecule, and a botulinum toxin, and a pharmaceutically acceptable diluent for injection.

In the above-mentioned therapeutic indications, the skeletal muscle cell is the postsynaptic cell.

Another object of the invention is a pharmaceutical composition for use in treating spasmodic dysphonia, laryngeal dystonia, oromandibular dysphonia, lingual dystonia and other voice disorders, achalasia, dysphagia, esophagia, gastroparesis, spasmodic colitis, neurogenic bladder, overreactive bladder, interstitial cystitis, benign prostatic hyperplasia, urinary dysfunction, fecal incontinence, constipation, anismus, anal fissure, , uterine pain (dysmenorrhea, dyspareunia), vaginal pain (vaginismus, vulvodynia), pelvic pain, ischiocavernous muscle (priapism), and other muscle tone disorders and other disorders characterized by involuntary movements of muscle groups, said composition comprising at least one postsynaptic inhibitor of cholinergic neuronal transmission, i.e. postsynaptic peptide or postsynaptic small molecule, and a botulinum toxin, and a pharmaceutically acceptable diluent for injection.

In the above-mentioned therapeutic indications, the smooth muscle cell is the postsynaptic cell.

Another object of the invention is a pharmaceutical composition for use in treating atrial fibrillation, said composition comprising at least one postsynaptic inhibitor of cholinergic neuronal transmission, i.e. postsynaptic peptide or postsynaptic small molecule, and a botulinum toxin, and a pharmaceutically acceptable diluent for injection.

In the above-mentioned therapeutic indications, the cardiac muscle cell is the postsynaptic cell.

Another object of the invention is a pharmaceutical composition for use in treating lacrimation, hyperhidrosis (hand, foot and armpit), sialorrhea, excessive salivation, excessive gastrointestinal secretions, excessive production of sebaceous glands (and related conditions such as acne) and other secretory disorders, said composition comprising at least one postsynaptic inhibitor of cholinergic neuronal transmission, i.e. postsynaptic peptide or postsynaptic small molecule, and a botulinum toxin, and a pharmaceutically acceptable diluent for injection.

In the above-mentioned therapeutic indications, the secretory gland cell is the postsynaptic cell.

In a particular embodiment of these intended uses, the at least one postsynaptic inhibitor of cholinergic neuronal transmission is a fast-onset inhibitor.

In a more particular embodiment of these intended uses, the at least one postsynaptic inhibitor is a fast-onset inhibitor and the botulinum toxin is of type A, B or E, preferably of type A.

The invention also relates to a method for treating an individual in need of treatment with injectable botulinum neurotoxin, comprising administering a pharmaceutically effective dose of a pharmaceutical composition comprising at least one postsynaptic inhibitor of cholinergic neuronal transmission, i.e. postsynaptic peptide or postsynaptic small molecule, and a botulinum neurotoxin into an area of the individual in need of treatment to achieve an aesthetic or therapeutic effect. The invention is preferably suitable for use in treating a condition associated with unwanted activity of cholinergic neurons and its potentially associated pain, in a subject in need thereof.

In a particular embodiment, the aesthetic effect is reduction of wrinkles, lines, such as glabellar lines, or furrows, reduction of muscle volume for aesthetic purposes (such as masseter or calf), reduction of hypertrophic scars and treatment of other dermatological conditions.

In another particular embodiment, the therapeutic effect is reduction of movement disorder, dystonia, cervical dystonia, spasmodic torticollis, focal dystonia, focal hand dystonia, blepharospasm, eyelid disorder, strabismus, spasticity, cerebral palsy, focal spasticity, limb spasticity, spasms, hemifacial spasm, tremors, tics, bruxism, apraxia and freezing of gait.

In another particular embodiment, the therapeutic effect is reduction of spasmodic dysphonia, laryngeal dystonia, oromandibular dysphonia, lingual dystonia and other voice disorders, achalasia, dysphagia, esophagia, gastroparesis, spasmodic colitis, neurogenic bladder, overactive bladder, interstitial cystitis, benign prostatic hyperplasia, urinary dysfunction, fecal incontinence, constipation, anismus, anal fissure, reduction or alleviation of uterine pain (dysmenorrhea, dyspareunia), vaginal pain (vaginismus, vulvodynia), pelvic pain, ischiocavernous muscle (priapism), reduction of other muscle tone disorders and other disorders characterized by involuntary movements of muscle groups.

In another particular embodiment, the therapeutic effect is reduction of atrial fibrillation.

In another particular embodiment, the therapeutic effect is reduction of lacrimation, hyperhidrosis (hand, foot and armpit), sialorrhea, excessive salivation, excessive gastrointestinal secretions, excessive production of sebaceous glands (and related conditions such as acne) and other secretory disorders.

The therapeutic needs include treatment of conditions wherein activity of specific cell types has to be modulated. The composition of the invention makes such modulation possible.

In another particular embodiment, when the postsynaptic cell is a skeletal muscle cell, the condition to be treated is movement disorder, dystonia, cervical dystonia, spasmodic torticollis, focal dystonia, focal hand dystonia, blepharospasm, eyelid disorder, strabismus, spasticity, cerebral palsy, focal spasticity, limb spasticity, spasms, hemifacial spasm, tremors, tics, bruxism, apraxia and freezing of gait.

In another particular embodiment, when the postsynaptic cell is a smooth muscle cell, the condition to be treated is spasmodic dysphonia, laryngeal dystonia, oromandibular dysphonia, lingual dystonia and other voice disorders, achalasia, dysphagia, esophagia, gastroparesis, spasmodic colitis, neurogenic bladder, overactive bladder, interstitial cystitis, benign prostatic hyperplasia, urinary dysfunction, fecal incontinence, constipation, anismus, anal fissure, uterine pain (dysmenorrhea, dyspareunia), vaginal pain (vaginismus, vulvodynia), pelvic pain, ischiocavernous muscle (priapism), other muscle tone disorders and other disorders characterized by involuntary movements of muscle groups.

In another particular embodiment, when the postsynaptic cell is a cardiac muscle cell, the condition to be treated is atrial fibrillation.

In another particular embodiment, when the postsynaptic cell is a secretory gland cell, the condition to be treated is lacrimation, hyperhidrosis (hand, foot and armpit), sialorrhea, excessive salivation, excessive gastrointestinal secretions, excessive production of sebaceous glands (and related conditions such as acne) and other secretory disorders.

The invention also relates to the use of a composition comprising at least one postsynaptic inhibitor of cholinergic neuronal transmission and a botulinum neurotoxin, or a pharmaceutical composition comprising said composition, for the manufacture of a medicament for the treatment of :
- movement disorder, dystonia, cervical dystonia, spasmodic torticollis, focal dystonia, focal hand dystonia, blepharospasm, eyelid disorder, strabismus, spasticity, cerebral palsy, focal spasticity, limb spasticity, spasms, hemifacial spasm, tremors, tics, bruxism, apraxia and freezing of gait,
- spasmodic dysphonia, laryngeal dystonia, oromandibular dysphonia, lingual dystonia and other voice disorders, achalasia, dysphagia, esophagia, gastroparesis, spasmodic colitis, neurogenic bladder, overactive bladder, interstitial cystitis, benign prostatic hyperplasia, urinary dysfunction, fecal incontinence, constipation, anismus, anal fissure, uterine pain (dysmenorrhea, dyspareunia), vaginal pain (vaginismus, vulvodynia), pelvic pain, ischiocavernous muscle (priapism), other muscle tone disorders and other disorders characterized by involuntary movements of muscle groups,

- atrial fibrillation,
- lacrimation, hyperhidrosis (hand, foot and armpit), sialorrhea, excessive salivation, excessive gastrointestinal secretions, excessive production of sebaceous glands (and related conditions such as acne) and other secretory disorders.

A last object of the invention concerns a kit for implementing the method of the invention, wherein the kit comprises a needle and its corresponding syringe, or smaller needle and a corresponding syringe. Preferably, the needle is a 25 to 30 Gauge needle.

The skilled artisan is able to choose the size of the needle according to the site of injection of the composition of the invention.

The kit further comprises at least one postsynaptic inhibitor of cholinergic neuronal transmission, i.e. postsynaptic peptide or postsynaptic small molecule, having binding specificity for nicotinic acetylcholine receptors (nAChRs, in particular (α1)2β1δε or (α1)2β1δγ), M1, M2 or M3 muscarinic acetylcholine receptors (mAChRs), voltagedependent, L type calcium channel (CaV 1.1 and CaV1.2), large-conductance Ca2⁺ release channels known as ryanodine receptors (RyRs) and in particular RyR1 present in skeletal muscle or RYR2 present in smooth muscle or cardiac muscle, voltage-gated sodium channels (VGSCs) such as voltage-gated Na⁺ (NaV) channels, and in particular NaV1.4 and Nav1.5, α1- or β1-adrenergic receptors, and a botulinum neurotoxin, preferably a botulinum neurotoxin of type A, B or E. The at least one postsynaptic inhibitor of cholinergic neuronal transmission and the botulinum toxin are either mixed together in a vial (lyophilized or liquid), or presented in two chambers for immediate reconstitution before injection, or in two separate vials to be reconstituted and injected one after the other.

The following examples are provided to illustrate certain preferred embodiments and aspects of the present invention and are not to be construed as limiting the scope thereof.

### EXAMPLES

### Injectable Botulinum neurotoxin compositions

### BoNT/A composition

BoNT/A composition is an injectable formulation that contains type A botulinum neurotoxin (BoNT/A). Dried BoNT/A is reconstituted according to the manufacturer's instructions in saline solution and supplemented with 33% rat serum. The 150 KDa active botulinum toxin A XEOMIN^{®} from Merz Pharmaceuticals is used for the experiments. The tested dose of BoNT/A is 2.5 to 5 Units/kg, corresponding to approximately 10 to 20 pg/kg.

### Combo composition

Combo composition is an injectable formulation that contains type A botulinum neurotoxin (BoNT/A) and at least one postsynaptic inhibitor of cholinergic neuronal transmission. Depending on the nature of the post-synaptic neural transmission inhibitor, different protocols are used to develop combo compositions.

### 1.2.1 Combo composition with postsynaptic peptide

Dried BoNT/A is reconstituted according to the manufacturer's instructions in saline solution. Dried postsynaptic peptide is reconstituted in saline supplemented with rat serum and added to the BoNT/A reconstituted solution. Postsynaptic peptides are obtained by solid-phase peptide synthesis or purchased from supplier (α-Bungarotoxin is purchased from Abcam). Postsynaptic peptide dose used in the experiments ranges from 1 to 350 µg/kg.

### 1.2.2 Combo composition with postsynaptic small molecule

Dried BoNT/A is reconstituted according to the manufacturer's instructions in saline solution. Dried postsynaptic small molecule is reconstituted in saline, or water, or water/DMSO and can be supplemented with rat serum; postsynaptic small molecule is finally added to the BoNT/A reconstituted solution. Postsynaptic small molecules are purchased from supplier (pancuronium, dantrolene, amlodipine, nicardipine, verapamil and diltiazem are purchased from Abcam). Small molecule dose used in the experiments ranges from 1 to 1200 µg/kg.

### Experimental procedure

### • Digit abduction score (DAS)

### Animals

Experiments were carried out with adult Sprague-Dawley rats (200g or , 400g), obtained from Charles River/France, or Janvier Labs/France or Envigo/France. Rats were acclimatized for at least one week prior to use with access to food and water *ad libitum* in animal facility. Unless otherwise stated, 5 rats for each condition were employed. Other experiments were carried out with CD-1 mice (20g), that were obtained and treated as described above.

The body weight of animals is recorded all along the study on day 0, 2, 4, 7 and once a week until the end of experiment.

### Treatment

Animals were injected (10 µL or 20 µL per rat) using a 30-or33-gauge needle attached to a 100µL Hamilton Syringe. On the initial day of the experiment, before the injections, rodents were pre-screened for a "zero" DAS response. Then, rats received intramuscular (IM) injections into the right tibialis anterior (TA) muscle. The left TA muscle was injected with saline solution (10 µL) as control.

### Digit abduction score assay (DAS)

The muscle paralysis was measured using the digit score abduction (DAS) assay as reported by Broide et al.(TOXICON, 2013, 71,pp. 18-24). Animals were scored for DAS response at different time points following the compound injections by eliciting digit abduction: 5 minutes; 15 minutes; 30 minutes; 1 hour; 2 hours; 6 hours; 12 hours; 24 hours; 2 days, 3 days, 4 days, 5 days, 6 days, 7 days, 8 days and once au day until two consecutive measurements on two consecutive days are equal to zero (DAS = 0). The varying degrees of digit abduction were scored on a five-point scale (0 = normal to 4 = maximum reduction in digit abduction, i.e. none of the toes can abduct) by two separate observers who were blinded to the treatment.

As typically performed for rat DAS studies, the DAS response were induced by grasping the rat around the torso, lifting into the air and simulating a drop to a flat surface. The rat reflexively usually braces for impact by spreading the digits in its hind paws and the DAS response was immediately scored with the animal facing up in a reclining position.

### Data analysis

As described above, a DAS score of '1' was assigned when loss of abduction was observed with the 1st digit. A DAS score of '2' was given when three digits are coupled together. A score of '3' was given when four digits are conjoined, and rats received a score of '4' when all five digits of the right paw were grouped together during abduction.

The DAS responses at each time point are measured and kinetics (onset of muscle weakening and duration of efficacy) are assessed and compared in the different groups of rats.

For each rat, areas under the DAS curve (in DAS*hours units) were calculated using series of Riemann sums between T=0 and T=24 hours (early AUC), between T=24 and T= 120 hours (medium AUC) and between T=120 hours to the end of the experiment (late AUC).

For each group, the onset of action was defined as the first time with a mean DAS >1; the duration of action was defined as the last time point with a mean DAS >1; the peak of action was defined as the maximum mean DAS value.

### Action of a combination of BoNT/A with a postsynaptic peptide that binds voltage-gated sodium channel NaV1.4

To determine the pharmacodynamic profile of a combination of BoNT/A with a µ-conotoxin Cnlllc postsynaptic peptide compared to BoNT/A alone, rats were injected into the right tibialis anterior (TA) muscle with 5 U/kg of BoNT/A combined with µ-conotoxin Cnlllc postsynaptic peptide at doses ranging from 0.4 to 40 µg/kg, and with 5 U/kg of BoNT/A alone. The sequence of µ-conotoxin Cnlllc postsynaptic peptide used is this experiment is SEQ ID NO: 54.

The results in Figure 1 show an onset of action for BoNT/A at 12 hours post-injection, with a peak of action of 3 at 72 hours. The duration of action is at 5 days.

### Faster onset of action

Injection of the combinations of BoNT/A and the µ-conotoxin Cnlllc postsynaptic peptide results in a significantly shorter onset of action compared to that of BoNT/A alone. The onset of action is dependent on the dose of the µ-conotoxin Cnlllc: just after 1 hour at 40 µg/kg; just before 2 hours at 20 µg/kg, and around 4 hours at 8 µg/kg.

### Increased peak

The peak of action is increased from 3.4 for BoNT/A alone in to 4.0 in the presence of the highest dose of µ-conotoxin Cnlllc.

### Increased duration

The duration of action of the combinations is also longer than that of BoNT/A alone, and it is dependent on the dose of µ-conotoxin Cnlllc used: between 168 and 192 hours at 8 µg/kg, and between 192 and 216 hours at 20 µg/kg and between 216 and 240 hours at 40 µg/kg (vs. just over 120 hours for BoNT/A alone).

Altogether, these data indicate a faster onset of local myorelaxation, more potent myorelaxation effect, and increased duration by the combination of BoNT/A and µ-conotoxin Cnlllc, when compared to BoNT/A alone.

### Synergy

The enhancement of BoNT/A effect observed when combining BoNT/A with µ-conotoxin Cnlllc cannot be explained by the mere additive effect of both components of the composition. These results are shown in Figure 2.

To analyze the synergistic effect of the combination between BoNT/A and a µ-conotoxin Cnlllc postsynaptic peptide, the combination was compared to BoNT/A alone, and µ-conotoxin Cnlllc peptide alone. Groups of 5 rats were injected into the right tibialis anterior (TA) muscle with 5 U/kg of BoNT/A combined with µ-conotoxin Cnlllc postsynaptic peptide at dose of 80 µg/kg, with 5 U/kg of BoNT/A alone or with µ-conotoxin Cnlllc postsynaptic peptide at dose of 80 µg/kg. The sequence of µ-conotoxin Cnlllc postsynaptic peptide used is this experiment is SEQ ID NO: 54.

The results, shown in Figure 2, indicate that the peak DAS for BoNT/A appeared between 12 and 24 hours, with a maximum mean score of 2.8 at 72 hours post injection. DAS for BoNT/A started to decrease at day 4. Duration of the effect was observed until 6 days post BoNT/A injection. The onset of µ-conotoxin Cnlllc postsynaptic peptide was observed between 0.5 and 1 hour post injection, with a maximum intensity of 3.6 at 2 hours post injection. DAS for the µ-conotoxin Cnlllc peptide alone started to decrease as early as 6 hours post injection. Logically, the calculated addition of both treatments showed two peaks, the first at 2 hours and the second at 72 hours post injection; notably, the score between the two peaks comes back to low levels (DAS<2). Strikingly, the combination of BoNT/A with µ-conotoxin Cnlllc does not show two peaks, but rather a sustained myorelaxation, from 2-6 hours (onset) up to 144-168 hours (duration) post injection. More than an addition of the effects of both components, the combination of BoNT/A with postsynaptic µ-conotoxin Cnlllc exhibit a synergistic effect.

Calculations of area under the curves (AUC in DAS*hours units) illustrate the synergistic effect:

**Table 11: Areas under the curves (from Figure 2)**

| | | **Early AUC** | **Medium AUC** | **Late AUC** |
|---|---|---|---|---|
| 1. | BoNT/A | 61.1 | 180.3 | 3.0 |
| 2. | µ-conotoxin Cnlllc | 39.7 | 0.0 | 0.0 |
| 3. | BoNT/A + µ-conotoxin Cnlllc | 123.4 | 240.0 | 103.2 |
| 4. | Addition (1 plus 2) | 100.8 | 180.3 | 3.0 |
| 5. | Synergy (3 minus 4) | 22.6 | 59.7 | 100.2 |

The table demonstrates that the effect of the combination of BoNT/A and µ-conotoxin Cnlllc on the onset (early: 123.4 > 100.8), the intensity (medium: 240 > 180.3) and the duration (late: 103.2 > 3) of action is greater than the added effect of both components.

### Action of a combination of BoNT/A with a postsynaptic inhibitor of cholinergic neuronal transmission that binds nicotinic acetylcholine receptors (nAChRs)

This example compares the onset, intensity and duration of local myorelaxation in rats injected with either BoNT/A or a combination of BoNT/A with three postsynaptic inhibitors of neuronal transmission, all binding nAChRs. Only one rat per group was treated.

### Pancuronium

To determine the onset and duration of action of BoNT/A compared to a combination of BoNT/A with a pancuronium, rats were injected with 5 U/kg of BoNT/A or a combination of 5 U/kg of BoNT/A and 57 µg/kg of pancuronium.

The results, shown in Figure 3, indicate that the peak DAS for BoNT/A appeared between 12 and 24hours, with a maximum score of 3.2 at 72 hours post injection. DAS for BoNT/A started to decrease at day 4. Complete recovery of digit abduction occurs 7 days post BoNT/A injection. Injection of the combination of BoNT/A and pancuronium results in a faster onset in the DAS compared to injection with BoNT/A; the onset appeared between 5 and 15 minutes post injection. The maximum DAS score is also increased to 4.0 in the presence of pancuronium. Lastly, the duration of myorelaxation is slightly increased by the addition of pancuronium to BoNT/A; digit abduction is still impaired 6 days after injection (DAS=2), whereas BoNT/A-treated rats are starting to recover (DAS<1).

These data indicate a faster onset of local myorelaxation, more potent myorelaxation effect, and increased duration of action by BoNT/A and pancuronium combination compared to BoNT/A alone.

### α-conotoxin postsynaptic peptide

To determine the onset, intensity and duration of action of BoNT/A compared to a combination of BoNT/A with a Conus magus postsynaptic peptide, rats were injected with 5 U/kg of BoNT/A or a combination of 5 U/kg of BoNT/A and 8 µg/kg of α-conotoxin MI peptide of SEQ ID NO: 8.

The results, shown in Figure 3, indicate that the peak DAS for BoNT/A appeared between 12 and 24 hours, with a maximum score of 3.2 at 72 hours post injection. DAS for BoNT/A started to decrease at day 4. Complete recovery of digit abduction occurs 7 days post BoNT/A injection. Injection of the combination of BoNT/A and α-conotoxin MI peptide results in a faster onset in the DAS compared to injection with BoNT/A; onset appeared between 2 and 4 hours post injection. The maximum DAS score is also increased to 4.0 in the presence of α-conotoxin MI peptide. Lastly, the duration of myorelaxation is greatly increased by the addition of α-conotoxin MI peptide to BoNT/A; digit abduction is still not recovered 12 days post injection.

These data indicate a faster onset of local myorelaxation, more potent myorelaxation effect, and increased duration of action by BoNT/A and α-conotoxin MI peptide combination compared to BoNT/A alone.

### α-bungarotoxin

To determine the onset and duration of action of BoNT/A compared to a combination of BoNT/A with a Bungarus toxin peptide, rats were injected with 5 U/kg of BoNT/A or 5 U/kg of BoNT/A combined with 17 µg/kg of α-bungarotoxin peptide of SEQ ID NO: 4.

DAS were recorded for each rat at several time points throughout the first 10 minutes through 12 days (288 hours) post injection. The results, shown in Figure 3, indicate that the peak DAS for BoNT/A appeared between 12 and 24 hours, with a maximum score of 3.2 at 72 hours post injection. DAS for BoNT/A started to decrease at day 4. Complete recovery of digit abduction occurs 7 days post BoNT/A injection. Injection of the combination of BoNT/A and α-bungarotoxin results in a faster onset in the DAS compared to injection with BoNT/A; onset appeared between 15 and 30 minutes post injection. The maximum DAS is also increased to 4.0 in the presence of α-bungarotoxin. Lastly, the duration of myorelaxation is increased by the addition of α-bungarotoxin to BoNT/A by 4 days.

These data indicate a faster onset of local myorelaxation, more potent myorelaxation effect, and increased duration of action by BoNT/A and α-bungarotoxin combination compared to BoNT/A alone.

The overall data illustrate that a faster onset and/or a longer duration of effect and/or an increased effect of botulinum toxin may be obtained by using postsynaptic inhibitors of neuronal transmission targeting nAChR.

### Action of a combination of BoNT/A with a postsynaptic inhibitor of cholinergic neuronal transmission that binds Ryanodine receptor (RYR1)

### Dantrolene

To determine the onset and duration of action of BoNT/A compared to a combination of BoNT/A with dantrolene, a known inhibitor of RYR1, rats were injected with 5 U/kg of BoNT/A or 5 U/kg of BoNT/A combined with 1.6 µg/kg of dantrolene.

DAS were recorded for each rat at several time points throughout the first 10 minutes through 12 days (288 hours) post injection. The results, shown in Figure 4, indicate that the peak DAS for BoNT/A appeared between 12 and 24 hours, with a maximum score of 3.2 at 72 hours post injection. DAS for BoNT/A started to decrease at day 4. Complete recovery of digit abduction occurs 7 days post BoNT/A injection. Injection of the combination of BoNT/A and dantrolene results in a faster onset in the DAS compared to injection with BoNT/A; onset appeared between 2 and 4 hours post injection. The maximum DAS is also increased to 4.0 in the presence of dantrolene. Lastly, the duration of myorelaxation is greatly delayed by the addition of dantrolene to BoNT/A; digit abduction is still not recovered 12 days after injection.

These data indicate a faster onset of local myorelaxation, more potent myorelaxation effect, and increased duration of action by BoNT/A and dantrolene combination compared to BoNT/A alone.

### Insecticidal Toxin LaIT1

To determine the onset and duration of action of BoNT/A compared to a combination of BoNT/A with the Insecticidal Toxin LalT1, rats were injected with 5 U/kg of BoNT/A or 5 U/kg of BoNT/A and 36 µg/kg of LaIT1 peptide of SEQ ID NO: 23.

DAS were recorded for each rat at several time points throughout the first 10 minutes through 12 days (288 hours) post injection. The results, shown in Figure 4, indicate that the peak DAS score for BoNT/A appeared between 12 and 24 hours, with a maximum score of 3.2 at 72 hours post injection. DAS for BoNT/A started to decrease at day 4. Complete recovery of digit abduction occurs 7 days post BoNT/A injection. Injection of the combination of BoNT/A and Insecticidal Toxin LaIT1 results in a faster onset in the DAS compared to injection with BoNT/A; onset appeared between 2 and 6 hours post injection. The maximum DAS is also increased to 4.0 in the presence of Insecticidal Toxin LalT1. Lastly, the duration of myorelaxation is delayed by the addition of Insecticidal Toxin LalT1 to BoNT/A by 5 days. Thus a longer duration of action of BoNT/A is obtained.

These data indicate a faster onset of local myorelaxation, more potent myorelaxation effect, and increased duration of action by BoNT/A and Insecticidal Toxin LalT1 combination compared to BoNT/A.

The overall data illustrate that a faster onset and/or a longer duration of effect and/or an increased effect of botulinum toxin may be obtained by using postsynaptic inhibitors of neuronal transmission targeting RYR1.

### Action of a combination of BoNT/A with two closely related postsynaptic inhibitors of cholinergic neuronal transmission that binds voltage-gated sodium channel NaV1.4

The pharmacodynamic profile of a combination of BoNT/A with µ-conotoxin Cnlllc postsynaptic peptide was compared with the one of a combination of BoNT/A with a peptide variant of µ-conotoxin Cnlllc. The variant peptide used in this experiment has a sequence (SEQ ID NO: 95) matching the consensus sequence defined by SEQ ID NO: 53; the sequence of the wild-type µ-conotoxin Cnlllc peptide used is this experiment is SEQ ID NO: 54. For this comparison, rats were injected into the right tibialis anterior (TA) muscle with 5 U/kg of BoNT/A combined with each µ-conotoxin Cnlllc postsynaptic peptide at 25 µg/kg; control rats were injected with 5 U/kg of BoNT/A alone.

The results have been analyzed by calculating the Areas Under the Curve (AUC) during the early, mid and late phase of the effect; for each phase, the percent of AUC increase with respect to control (BoNT/A alone) are presented for the purpose of comparison. The final calculations, shown in Figure 5, first demonstrate that both combinations are able to increase the onset of action (early AUC), intensity of action (mid AUC) and duration of action (late AUC), all percentages being above 0%. Second, the results indicate that the combination with the variant exhibit a slightly different pharmacological profile compared to the combination with the wild-type µ-conotoxin Cnlllc: the former effects are more pronounced at the early and late phase, while the effect on the peak intensity is milder.

The overall data illustrate the advantage of categorizing postsynaptic peptide inhibitor into families sharing a consensus sequence. On the one hand, peptides within a family share similar effects; on the other hand, their slightly different pharmacological profiles can be advantageously exploited for drug design.

### Action of a combination of BoNT/A with a postsynaptic inhibitor of cholinergic neuronal transmission that binds Voltage-dependent, L type, calcium channel (Cav1.1)

### Amlodipine

To determine the onset and duration of action of BoNT/A compared to a combination of BoNT/A with amlodipine, a known inhibitor of Cav1.1, rats were injected with 5 U/kg of BoNT/A or 5 U/kg of BoNT/A combined with 83 µg/kg of amlodipine.

DAS were recorded for each rat at several time points throughout the first 10 minutes through 14 days (336 hours) post injection. The results, shown in Figure 6, indicate that the peak DAS for BoNT/A appeared between 24 and 48 hours, with a maximum score of 3.8 at 72 hours post injection. DAS for BoNT/A started to decrease at day 4; the scores were above 1 until day 11. Injection of the combination of BoNT/A and amlodipine results in a faster onset in the DAS compared to injection with BoNT/A; onset appeared between 2 and 4 hours post injection. The maximum DAS is also increased to 4.0 in the presence of amlodipine. Lastly, the duration of myorelaxation is delayed by the addition of amlodipine to BoNT/A; digit abduction scores are maintained above 1 until day 13.

These data indicate a faster onset of local myorelaxation, more potent myorelaxation effect, and increased duration of action by BoNT/A and amlodipine combination compared to BoNT/A alone.

### Diltiazem

To determine the onset and duration of action of BoNT/A compared to a combination of BoNT/A with diltiazem, a known inhibitor of Cav1.1, rats were injected with 5 U/kg of BoNT/A or 5 U/kg of BoNT/A combined with 83 µg/kg of diltiazem.

DAS were recorded for each rat at several time points throughout the first 10 minutes through 14 days (336 hours) post injection. The results, shown in Figure 6, indicate that the peak DAS for BoNT/A appeared between 24 and 48 hours, with a maximum score of 3.8 at 72 hours post injection. DAS for BoNT/A started to decrease at day 4; the scores were above 1 until day 11. Injection of the combination of BoNT/A and diltiazem results in a faster onset in the DAS compared to injection with BoNT/A; onset appeared between 0.5 and 1hour post injection. The maximum DAS is also increased to 4.0 in the presence of diltiazem. Lastly, the duration of myorelaxation is delayed by the addition of diltiazem to BoNT/A; digit abduction scores are maintained above 1 until day 13.

These data indicate a faster onset of local myorelaxation, more potent myorelaxation effect, and increased duration of action by BoNT/A and diltiazem combination compared to BoNT/A alone.

### Verapamil

To determine the onset and duration of action of BoNT/A compared to a combination of BoNT/A with verapamil, a known inhibitor of Cav1.1, rats were injected with 5 U/kg of BoNT/A or 5 U/kg of BoNT/A combined with 83 µg/kg of verapamil.

DAS were recorded for each rat at several time points throughout the first 10 minutes through 14 days (336 hours) post injection. The results, shown in Figure 6, indicate that the peak DAS for BoNT/A appeared between 24 and 48 hours, with a maximum score of 3.8 at 72 hours post injection. DAS for BoNT/A started to decrease at day 4; the scores were above 1 until day 11. Injection of the combination of BoNT/A and verapamil results in a faster onset in the DAS compared to injection with BoNT/A; onset appeared between 4 and 6 hours post injection. The maximum DAS is also increased to 4.0 in the presence of verapamil. Lastly, the duration of myorelaxation is comparable to the duration of BoNT-induced myorelaxation.

These data indicate a faster onset of local myorelaxation and more potent myorelaxation effect by BoNT/A and verapamil combination compared to BoNT/A alone.

### Action of a combination of BoNT/A with a small molecule which is a postsynaptic inhibitor of cholinergic neuronal transmission: comparisons

To determine the pharmacological profile of BoNT/A compared to a combination of BoNT/A with a small molecule which is a postsynaptic inhibitor, rats were injected with 5 U/kg of BoNT/A or 5 U/kg of BoNT/A combined with various doses of several small molecules. These molecules were known inhibitors of nAChR (pancuronium, suxamethonium), or RYR1 (dantrolene), or Cav1.1 (Amlodipine, Diltiazem, Verapamil). The results have been analyzed by calculating the Areas Under the Curve (AUC) during the course of the experiment; the percent of AUC increase with respect to control (BoNT/A alone) are presented for the purpose of comparison.

The results, shown in Figure 7, demonstrate that all tested small molecules are able to enhance the effect of BoNT myorelaxation. The effect of the combination of dantrolene and BoNT is especially remarkable.

### Action of a combination of BoNT/A with a peptide which is a postsynaptic inhibitor of cholinergic neuronal transmission: comparisons

To determine the pharmacological profile of BoNT/A compared to a combination of BoNT/A with a peptide which is a postsynaptic inhibitor, rats were injected with 5 U/kg of BoNT/A or 5 U/kg of BoNT/A combined with various doses of several peptides. These molecules were known inhibitors of nAChR (α-Bungarotoxin, α-conotoxin MI, Waglerin-1, αC-Conotoxin PrXA), or RYR1 (LaIT1 toxin, Imperacalcin), or Cav1.1 (U7-Ctenitoxin Pn1b, ω-conotoxin TxVll, U6-Ctenitoxin Pnla, U9-Ctenitoxin Pnla, κ-Ctenitoxin Pnla and Glacontryphan), or Nav1.4 (µ-conotoxin Cnlllc, µ-conotoxin Glllb, µ-Thomitoxin Hmela, µ-conotoxin GvllJ, µO-conotoxin MfVIA and µ-Thomitoxin Hmelb). The results have been analyzed by calculating the Areas Under the Curve (AUC) during the course of the experiment; the percent of AUC increase with respect to control (BoNT/A alone) are presented for the purpose of comparison.

The results, shown in Figure 8, demonstrate that all tested small molecules are able to enhance the effect of BoNT myorelaxation. The effect of the combination of α-Conotoxin MI and BoNT is especially remarkable.

### • Compound Muscle Action Potential (CMAP)

### Animals

Experiments were carried out with adult Sprague-Dawley rats (200g), obtained from Charles River, France or Janvier Labs, France or Envigo, France. Rats were acclimatized for at least one week prior to use with access to food and water *ad libitum* in animal facility.

The body weight of animals is recorded every day all along the study until the end of experiment.

### Data acquisition and treatment

### - Treatment

Animals were injected (20 µL per rat) using a 33-gauge needle attached to the 100µL Hamilton Syringe. Then, rats received intramuscular (IM) injections into the right tibialis anterior (TA) muscle.

### - Anesthesia

The animals are anesthetized with isoflurane for each CMAP measurement. The animals are maintained on heating plate at 37°C to control the body temperature and hydrated with physiological saline solution (by subcutaneous injection) before anesthesia.

### - Measurement

The animal is placed on the heating pad in prone position for measurement. The electrode needles for CMAP measurement are placed as follows:
- Simulating electrodes are placed subcutaneously on the both side of sciatic notch.
- Recording electrode is placed subcutaneously, aligned with the *tibialis anterior.*
- Reference electrode is placed subcutaneously next to the Achille tendon.
- Ground electrode is place subcutaneously on opposite side of simulating electrodes.

The CMAP measurement is realized and recorded by stimulation with the Natus^{®} UltraPro S100 EMG device.

Typically, this measure is realized 6 days before the injection for the baseline measurement and at different time points following the compound injection: 2 hours, 1 day, 2 days, 4 days, 7 days, 9 days, 11 days, and 14 days.

### Data analysis

Two parameters are analyzed during the measurement: the latency and peak-to-peak amplitude. The latency is determined by the delay from the stimulation to the onset of the CMAP response. The peak-to-peak amplitude is measured from the maximum negative to the maximum positive peak of the biphasic wave. CMAP amplitude can then be plotted in function of time of measurement. On this curve, onset of action is defined as the time when inhibition becomes greater than 20% of the baseline; peak of activity is defined as the maximal inhibition (in percent of baseline); duration of action is defined as the time required to recover to 20% of the baseline.

All the data are compared group by group with a Student's t-test and homogeneity is compared with a Fisher's test.

### • In vivo Model of Neurogenic Detrusor Overactivity

### Animals

Experiments were carried out with adult Sprague-Dawley rats (200g), obtained from Charles River, France or Janvier Labs, France or Envigo, France. Rats were acclimatized for at least one week prior to use with access to food and water ad libitum in animal facility. After this period, the animals underwent a T8-T9 spinal cord trans-section. The body weight of animals is recorded every day all along the study until the end of experiment.

### Treatment

At 19 days post-spinalization, when the neurogenic detrusor overactivity is well-established, the animals are anesthetized under isoflurane and bladders are exposed and emptied. Using a microscope, the detrusor is injected using a 30G needle, connected to a Hamilton syringe with the solution. The injected dose is distributed in 4 or 8 injection sites avoiding the trigone. Then a catheter is inserted in the dome of the bladder, tunneled subcutaneously, externalized to the back of the neck and sutured between the shoulder blades. Postoperatively, the rats were treated with gentamicin.

### Measurement

Cystometry was performed in rats at different time points hours after intradetrusor injections : 24h, 48h, 7 days and 14 days.

First, the voiding and filling parameters were analyzed: maximal pressure of voiding contraction (millimeter of mercury mmHg), infused volume (bladder capacity in µL) and voiding efficiency (% ratio of voided volume to infused volume). Then, nonvoiding contraction (contractions with amplitude > 3mmHg during the filling phase) were analyzed: amplitude (mmHg), frequency (number per minute) and volume threshold to elicit nonvoiding phase (% of total filling volume).

### Data analysis

All data were expressed as mean +/- standard error of the mean per treatment group. A Grubb's test is used for exclusion of outliers. For each cystometry parameter, an analysis of variance was performed to compare the groups to allow their aggregation.

### • Ex vivo : bladder model

### Animals

Experiments were carried out with adult Sprague-Dawley rats (200g), obtained from Charles River, France or Janvier Labs, France or Envigo, France. Rats were acclimatized for at least one week prior to use with access to food and water ad libitum in animal facility. On the day of experiments, rats were anesthetized with isoflurane and exsanguinated before tissue collection. The bladder is collected and cleaned and two strips measuring about 6 x 2 mm are cut out from one bladder, fixed onto a custom electrode tissue holder, and tensed to 0.5g in organ baths, typically filled with KHB and bubbled with carbogen at 37°C and pH of 7.4. Bladder contraction force is measured with isometric transducers. After about 45 minutes of equilibrium period, with renewal of the buffer every 15 minutes, a verification of integrity of bladder are realized by applied KCI (Typically at 70mmol/L). A solution of carbachol (which activated acetylcholine receptors) is applied to test postsynaptic receptors (typically at 10µmol/L). Subsequently, contractions of the detrusor muscle are evoked by electrical field stimulation using train pulses. This stimulation was generated with the use of two platinum electrodes placed a few millimeters on each side of the strip.

### Treatment

After intensive washings and a period with stable contractions, treatment solutions are added in baths. Treatments contain various concentrations of BoNT/A, alone or mixed with postsynaptic inhibitors. Each strip is exposed to only one treatment and signals are recorded until 90% of the signal are abolished, or for 6 hours if this value is not reached. At the end of the experiment, a final addition of carbachol was performed to assess the viability of the tissue. Experiments were rejected when poststimulation carbachol responses were <80% of the prestimulation carbachol response.

### Data analysis

The results are expressed as a percentage of the initial control contraction just prior to addition of treatment. Data are expressed as individual data or as mean +/- standard error of the mean. Concentration curves were plotted for each group conditions. For statistical analysis, an unpaired Student's t test was performed.

## Claims

1. A method for enhancing the effect of a botulinum neurotoxin composition, comprising the step of adding at least one postsynaptic inhibitor of cholinergic neuronal transmission to the botulinum neurotoxin composition.

2. The method of claim 1, wherein enhancing the effect of the botulinum neurotoxin composition is accelerating the onset of action and /or extending the duration of action and/or enhancing the intensity of action of the botulinum neurotoxin composition.

3. The method of claim 1 or 2, wherein the onset of action of the botulinum neurotoxin composition comprising the at least one postsynaptic inhibitor of cholinergic neuronal transmission occurs 50%, preferably 75%, more preferably 90% earlier as compared to the onset of action of a botulinum neurotoxin composition not comprising any postsynaptic inhibitor of cholinergic neuronal transmission.

4. The method of any one of claims 1 to 3, wherein the duration of action of the botulinum neurotoxin composition comprising the at least one postsynaptic inhibitor of cholinergic neuronal transmission is extended by 10%, 25%, 50%, 100% or more as compared to the duration of action of a botulinum neurotoxin composition not comprising any postsynaptic inhibitor of cholinergic neuronal transmission.

5. The method of any one of claims 1 to 4, wherein the intensity of action of the botulinum neurotoxin composition comprising the at least one postsynaptic inhibitor of cholinergic neuronal transmission is enhanced by at least 2%, preferably at least 5%, more preferably at least 10% as compared to the maximum intensity of action of a botulinum neurotoxin composition not comprising any postsynaptic inhibitor of cholinergic neuronal transmission.

6. A composition suitable for implementing the method of any one of claims 1 to 5, the composition comprising at least one postsynaptic inhibitor of cholinergic neuronal transmission and a botulinum neurotoxin.

7. The composition of claim 6, wherein the at least one postsynaptic inhibitor of cholinergic neuronal transmission specifically binds to at least one receptor expressed by a skeletal muscle cell, said receptor being selected from the group comprising or consisting of (α1)₂β1δε nAChr, (α1)₂β1δγ nAChr, RyR1, CaV1.1 and Nav1.4.

8. The composition of claim 6, wherein the at least one postsynaptic inhibitor of cholinergic neuronal transmission specifically binds at least one receptor expressed by a smooth muscle cell selected from the group comprising or consisting of M3 mAChr, RyR2, CaV1.2 and Nav1.5.

9. The composition of claim 6, wherein the at least one postsynaptic inhibitor of cholinergic neuronal transmission specifically binds to at least one receptor expressed by a cardiac muscle cell, said receptor being selected from the group comprising or consisting of M2 mAChr, RyR2, CaV1.1, CaV1.2 and Nav1.5.

10. The composition of claim 6, wherein the at least one postsynaptic inhibitor of cholinergic neuronal transmission specifically binds at least one receptor expressed by a secretory gland cell, said receptor being selected from the group comprising or consisting of M1 mAChR, M3 mAChR, α₁ adrenergic receptor and β1 adrenergic receptor.

11. The composition of any one of claims 6 to 10, wherein the at least one postsynaptic inhibitor of cholinergic neuronal transmission is a postsynaptic peptide and/or a postsynaptic small molecule.

12. The composition of any one of claims 6 to 11, wherein the at least one postsynaptic inhibitor of cholinergic neuronal transmission is α-Conotoxin MI, µ-conotoxin Cnlllc, or derivatives thereof, pancuronium, dantrolene, or a combination thereof.

13. The composition of any one of claims 6 to 12, wherein the at least one postsynaptic inhibitor of cholinergic neuronal transmission is a fast onset postsynaptic peptide and/or fast onset postsynaptic small molecule.

14. The composition of any one of claims 6 to 12, wherein the at least one postsynaptic inhibitor of cholinergic neuronal transmission is compatible with the late onset of botulinum neurotoxin.

15. The composition of any one of claims 6 to 14, wherein the botulinum neurotoxin is type A, B, E, or a combination of heavy and light chains of type A, B, E botulinum neurotoxin.

16. The composition of any one of claims 6 to 15 for use in aesthetic treatment such as reducing wrinkles, lines, such as glabellar lines, or furrows, muscle volume for aesthetic purposes (such as masseter or calf), hypertrophic scars and other dermatological conditions.

17. The composition of any one of claims 6 to 15 for use in the treatment of (i) skeletal muscle disorders comprising movement disorder, dystonia, cervical dystonia, spasmodic torticollis, focal dystonia, focal hand dystonia, blepharospasm, eyelid disorder, strabismus, spasticity, cerebral palsy, focal spasticity, limb spasticity, spasms, hemifacial spasm, tremors, tics, bruxism, apraxia and freezing of gait and (ii) of the pain associated to these disorders.

18. The composition of any one of claims 6 to 15 for use in the treatment of (i) smooth muscle disorders comprising spasmodic dysphonia, laryngeal dystonia, oromandibular dysphonia, lingual dystonia and other voice disorders, achalasia, dysphagia, esophagia, gastroparesis, spasmodic colitis, neurogenic bladder, overreactive bladder, interstitial cystitis, benign prostatic hyperplasia, urinary dysfunction, fecal incontinence, constipation, anismus, anal fissure, , uterine pain (dysmenorrhea, dyspareunia), vaginal pain (vaginismus, vulvodynia), pelvic pain, ischiocavernous muscle (priapism), other muscle tone disorders and other disorders **characterized by** involuntary movements of muscle groups and (ii) of the pain associated to these disorders.

19. The composition of any one of claim 6 to 15 for use in the treatment of cardiac muscle cell disorder comprising atrial fibrillation.

20. The composition of any one of claims 6 to 15 for use in the treatment of secretory gland disorder comprising lacrimation, hyperhidrosis of hand, foot and armpit, sialorrhea, excessive salivation, excessive gastrointestinal secretions, excessive production of sebaceous glands, acne, and secretory disorders.

21. The composition of any one of claims 6 to 15 for use as disclosed in claims 16 to 20, wherein the onset of action is accelerated and/or the duration of action is extended and/or the intensity of action is enhanced as compared to a botulinum neurotoxin composition not comprising any postsynaptic inhibitor of cholinergic neuronal transmission.

22. The composition of claim 6, wherein the at least one postsynaptic inhibitor of cholinergic neuronal transmission (PoNT) and a botulinum neurotoxin are administered in combination, with the proviso that the botulinum neurotoxin is administered before the decay of PoNT activity occurs.
